# EUROPEAN PATENT APPLICATION

(11) **EP 4 174 490 A1**
(43) Date of publication of application: **03.05.2023**
(21) Application number: 21204729.4
(22) Date of filing: 26.10.2021
(51) Int. Cl.: G01N 33/52, G01N 33/53, G01N 33/543

(54) **IMMOBILIZATION OF AMINO GROUP-CONTAINING OR THIOL GROUP-CONTAINING BAIT MOLECULES ONTO IRON-BASED PARTICLES**

(71) Applicant: Friedrich-Alexander-Universität Erlangen-Nürnberg, 91054 Erlangen (DE)
(72) Inventor: The designation of the inventor has not yet been filed
(74) Representative: Wichmann, Hendrik

(57) **Abstract**

The present invention is concerned a method for identifying a protein of interest in an aqueous or body fluid sample. The identification is carried out by using a complex comprising (A) a superparamagnetic iron-based particle, (B) one or more catechol acid moieties, and (C) one or more bait molecules, wherein B has been bound to A, and wherein B has been covalently linked to C by activating the catechol acid and reacting said activated catechol acid with the amino group or the thiol group of C. The invention also relates to said complex as well as a use of said complex for identifying a protein of interest in an aqueous or body fluid sample. Finally, the invention also refers to a method for diagnosing a disease or disorder, wherein a protein of interest is identified in a body fluid sample of a patient by using said complex.

## Description

### Background of the invention

The stable binding of small molecules as well as large macromolecules to surfaces of nano-and microparticles is an important technique for therapeutic (e.g. drug delivery), diagnostic as well as other applications. For this purpose, iron oxide nanoparticles possess a unique versatility of attachment compared to other carriers or carrier-free applications [18]. In addition, the ability to be magnetically controllable is extremely advantageous for these particles, as washing steps and analyte concentration measures can be significantly simplified compared to conventional non-magnetic beads.

For example, the assessment of antibody status under the conditions of the Sars-CoV2 pandemic is of great importance for epidemiological evaluation. This is true for both: recording the extent to which infection has progressed and for surveying immunization status as the population vaccination campaign progresses. While a number of diagnostic methods exist, they are often device-specific. For example, the Cobas Elecsys Anti-SARS-CoV-2 chemiluminescence immunoassay as discussed in Lippi et al. [19] is based on a sandwich reaction, wherein the test sample, biotinylated SARS-CoV-2-specific recombinant antigen, and SARS-CoV-2-specific recombinant antigen labeled with ruthenium are initially incubated altogether. After adding streptavidin-coated microparticles, the complex is bound to the solid phase through a biotin-streptavidin reaction and then aspirated into a measuring cell, where microparticles are magnetically captured. Hence, such an assay is limited to assay-specific equipment and assay-specific disposals and adjustments relating to the binding partners cannot be easily implemented. Moreover, it is questionable whether existing capacity is sufficient to meet the upcoming demand for antibody screening. The other category of assessing antibody status are Point of Care (POC) diagnostics, which is based on a test strip technology, which represent an immunological rapid detection of antigens immobilised on solid phases and possible antibodies in the sample material. Here, a detailed insight is even less precise and the statement can only be made according to a simple yes-no scheme. Thus, there is a need in the art for new, reliable and widely applicable methods to identify proteins of interest such as the antibody status of a patient.

The use of flow cytometry is very suitable in terms of versatility and widespread use in clinics and, with nanoparticles as carriers for the analytes, offers excellent application potential. For this purpose, iron oxide nanoparticles have a unique versatility of attachment compared to other carriers or carrier-free applications. The surface with its specific iron-hydroxide arrangement offers the possibility that certain substances can attach very stably there [1]. Carboxyl groups, phosphates, phosphonates, or various polymers with hydroxyl groups or other functional groups are described in the literature, which contribute to stable coatings [20, 21]. In the case of OH groups, their large number in polymers is the main reason for stable additions. However, coating with small molecules is much more challenging, as it is subject to ligand exchange reactions under certain conditions. This is especially true for carboxyl groups. Stable coatings are achieved with small molecules only if several carboxyl groups act simultaneously on the particle surface [2, 3]. Phosphonates are considered as highly stable anchor groups as they form strong complexes with the iron hydroxide surface [1]. The availability and possibility of derivatization of these phosphonates is unfortunately limited, which is why further functionalization for versatile applications has been challenging so far.

Widely used, for example, are coating strategies with dopamine, which, however, tends to auto-polymerize under certain circumstances [6] and thus complicates further functionalization. In addition, dopamine has only one amino group for further functionalization with possible target molecules, which is homologous to the amino groups of possible targets and is therefore only suitable to a limited extent for corresponding binding to such groups. For example, catechols have been described to have the ability to generate firm complexes with the iron hydroxide surface of the corresponding particles [4, 5].

Hence, there is a need in the art for an optimized and robust linkage between the nanoparticle and a bait molecule, which can then reliable test for a protein of interest in a sample, e.g. the antibody status in a serum sample.

### Summary of the invention

The invention relates to a method for identifying a protein of interest in an aqueous or body fluid sample by using a complex comprising (A) a superparamagnetic iron-based particle, (B) one or more catechol acid moieties, and (C) one or more bait molecules, as further defined in the claims. Said complex allows a simple and robust detection of proteins of interest in an aqueous or body fluid sample. In these complexes, bait proteins, such as antigens (e.g. virus proteins), can be stably exposed on iron oxide-based nanoparticles. Proteins of interest, such as antibodies, can then be selectively detected upon binding to the bait protein and e.g. recorded in a flow cytometer. Thus, a versatile diagnostic platform is obtained, which can be used to detect protein of interest such as pathogens or autoantibodies, if a corresponding selective complementary binding partner, such as an antigen-antibody pair, exists.

The method is widely applicable, as it involves stochastic binding of the (A) superparamagnetic iron-based particle with (B) catechol acid to exposed amino or thiol groups in the (C) bait protein. By using this method, a wide variety of antigens can be covalently linked to the superparamagnetic iron-based particles as bait molecules. Subsequently, a protein of interest, which is a complementary binding partner to the bait protein, can be detected. Using a detection molecule such as a fluorescent dye-labelled antibody directed against the protein of interest, the presence of the protein of interest can be reliably determined e.g. by flow cytometer. The main purpose of application is *in vitro* diagnostics.

In this invention, activated catechols have been found to be especially advantageous, particularly when the catechols contain a carboxyl group, such as those found in derivatives of 3,4-dihydroxycinnamic acid, also known by the trivial name caffeic acid. This is because catechols have high affinities to metal oxide surfaces. Furthermore, many catechols are derived from known natural products (such as dopamine or caffeic acid) and are therefore well characterized and mostly non-toxic. Another example of carboxyl catechols is protocatechuic acid (3,4-dihydroxybenzoic acid). Caffeic acid has an acid group that can form a stable bond with corresponding nucleophiles. However, the acid group of catechol acid is generally not reactive enough for this reaction to occur under conditions that do not affect the nature of the particle system. The acid groups can be activated accordingly, but then the properties of the linker may be altered in such a way that the colloidal stability of the particle is no longer guaranteed. The activation of the catechol acid has the particular advantage that said activation is fully compatible with the binding to superparamagnetic iron-based particles and that the activated catechol acid readily reacts with exposed amino groups or via addition reaction of the unsaturated α,β position to the carboxyl with a thiol group on bait molecules. Specifically, activated catechol acid can be (i) readily bound to superparamagnetic iron-based particles by e.g. ligand replace reactions and (ii) covalently bound to amino or thiol groups of bait molecule, such as the Sars-CoV-2 antigen.

For example, the inventors have activated caffeic acid with pentafluorophenyl trifluoroacetate, resulting in the reactive pentafluorophenol caffeic acid ester (CAF-PFP) **(****Fig.** 1). Furthermore, the inventors have covalently bound the Sars-CoV-2 antigen (Spike protein) as well as and HIV-envelope protein as bait proteins to the activated catechol acid. As a proof of principle, the inventors show a diagnostic particle system for the selective detection of e.g. Sars-CoV2 antibodies from patient blood, which has proven its performance in samples of Covid-19 of convalescents. Hence, the data revealed a correct determination of patient sera having a positive or negative Sars-CoV2 antibody status **(****Fig.** 10). Similarly, the presence of anti-gp120 antibodies in a sample could be readily detected by using the envelope protein gp120 as a bait protein **(****Fig.** 11) .

### Detailed description of the invention

In a first aspect, a method for identifying a protein of interest in an aqueous or body fluid sample, the method comprising the steps of:
a) providing a complex comprising
   (A) a superparamagnetic iron-based particle,
   (B) one or more catechol acid moieties, and
   (C) one or more bait molecules,
      wherein B has been bound to A; and wherein B has been covalently linked to C by activating the catechol acid (benzene-1,2-diol acid) and reacting said activated catechol acid with an amino group or a thiol group of C;
b) contacting the complex of step a) with the sample whereby the bait molecule (C) binds to the protein of interest;
c) immobilizing the complex binding to the protein of interest of step b) by a magnetic field and thereby separating the protein of interest from the sample,
d) contacting the complex binding to the protein of interest of step c) with a detection molecule, wherein the detection molecule binds to the protein of interest; wherein the detection molecule (i) comprises a detectable label or (ii) is detectable by a labelling molecule comprising the label,
e) determining the presence of the label, thereby identifying the protein of interest.

In general, the methods as disclosed herein are *in vitro* methods. In other words, said methods are performed *ex vivo,* in particular outside of the human body.

As noted above, the present disclosure is concerned with a particularly advantageous linkage between the superparamagnetic iron-based particle (A) and the bait molecule (C). In general, A is connected to C via one or more catechol acid moieties (B). In particular, B has been bound to A.

In general, the term 'catechol acid moiety' as used herein refers to a catechol acid portion, which is bound by A and C. In other words, an activated catechol acid being bound to A and C is a catechol acid moiety. In an especially preferred embodiment, the superparamagnetic iron-based particle (A) is linked to the one or more catechol acid moieties via citrate. For exemplary data, reference is made to **Example 1** and **Table 1.** According to this disclosure, B has been covalently linked to C by activating the catechol acid (benzene-1,2-diol acid) and reacting said activated catechol acid with an amino group or thiol group of C. For example, the inventors have demonstrated such a linkage in **Example 1 (Table 1).** In said exemplary case, the bait molecule has been the SARS-CoV 2 Spike protein. As an additional proof of concept, the envelope protein gp120 of HIV has been used as a bait molecule in

### Example 2.

In particular, the catechol acid may comprise, preferably comprises, a carboxyl group, preferably wherein the catechol acid is caffeic acid (3,4-dihydroxycinnamic acid) or protocatechuic acid, more preferably wherein the catechol acid is caffeic acid. Caffeic acids has also been used in Examples 1 and 2 herein. However, the skilled person is able to determine other suitable catechol acids such as L-Dopa (L-3,4-dihydroxyphenylalanine), dihydroxybenzoic acid, dihydroxyindole-2-carboxylic acid. Preferably, the catechol acid has been activated to be a pentafluorophenyl catechol acid, terafluorophenyl catechol acid, or a catechol acid N-Hydroxysuccinimide ester, preferably wherein the catechol acid has been activated to be a pentafluorophenyl catechol acid, such as demonstrated in **Example 1** herein.

In general, the skilled person the skilled person is provided with several options to activate the catechol acid with. For example, the catechol acid may have been activated with a isocyanate, acyl azides, a sulfonyl chloride, a tosylate ester, an epoxide, an oxiranes, an imido ester, a carbodiimide, a fluorophenyl ester, or a O-methyl isourea. In case the catechol acid is caffeic acid, which is preferred, the caffeic acid may have been activated to be a pentafluorophenyl caffeic acid, terafluorophenyl caffeic acid, or a caffeic acid N-Hydroxysuccinimide ester. In an especially preferred embodiment, the catechol acid has been activated by pentafluorophenyl trifluoroacetate. It is further preferred that the catechol acid is caffeic acid and wherein pentafluorophenol caffeic acid ester has been formed by the activation of caffeic acid. In addition, the catechol acid may be caffeic acid and the caffeic acid may have been activated by pentafluorophenyl trifluoroacetate. Further assistance regarding the exact reaction conditions can be found in **Example 1.**

As noted above, the particular advantage of this linkage between the iron-based particles and the bait molecule is that a linkage via a catechol moiety provides a versatile tool for linking bait molecules to the particles reliably. This is, for example, because the bait molecule merely requires an amino group or a thiol group. In addition, activated catechol groups are highly reactive with bait molecules such as proteins. Furthermore, activated catechol are easy to handle and usually cheap in comparison to other alternatives. Catechols have the property that they polymerize with one another, thus adhering more stably to the particle surface and at the same time do not lose their ability to bind to the bait molecules via the activated ester. In addition, the described linkage can be combined with any bait molecule - protein of interest pair, as long as the bait molecule comprises an amino group or a thiol groups. Thus, the described linkage harbors a broad applicability making said linkage a versatile toolkit.

According to this disclosure, the protein of interest and the bait molecule generally have a high affinity to each other, such as a nanomolar affinity. The skilled person is aware of standard methods to determine the strength of macromolecular interactions, such as protein-protein interactions. For example, the skilled person may use SPR, electromobility shift assays, ITC, fluorescent polarization. Hence, particularly preferred are antibody-antigen interactions between the protein of interest and the bait molecule. It is preferred that the bait molecule comprises a binding site for the protein of interest, more preferably wherein the bait molecule provides an antigen for the protein of interest and wherein the protein of interest is an antibody.

The term 'protein of interest' refers to any protein, which may be part of an aqueous or body fluid sample. In the context of the bait molecule (C), the protein of interest may also be regarded as the 'prey', which is sought after by the bait protein. In particular, proteins of interest may be antibodies or pathogen proteins. Such a pathogen protein may be a viral protein, a bacterial protein, a fungal protein, or a protozoan protein. In general, the skilled person is aware of such proteins and which diseases such proteins of interest are indicative for. For example, the viral protein may be from an enveloped or a non-enveloped virus. Preferably the viral protein is from an enveloped virus. More specifically, the enveloped virus may be a coronavirus, retrovirus, herpesvirus, poxvirus, hepadnavirus, african swine fever and related viruses, flavivirus, alphavirus, togavirus, hepatitis D virus, orthomyxovirus, paramyxovirus, rhabdovirus, bunyavirus, or filovirus. Even more in particular, the enveloped virus may be a coronavirus, preferably a orthocoronavirus, more preferably a betacoronavirus, even more preferably a sarbecovirus, even more preferably a SARS-coronavirus or a MERS-coronavirus, even more preferably a SARS coronavirus, and most preferably SARS-coronavirus 2. Alternatively, the virus protein may be from a retrovirus such as HIV. It is preferred that the retrovirus is an orthoretrovirus, preferably a lentivirus, more preferable human immunodeficiency viruses (HIV). As a further alternative, the virus protein may be from a non-enveloped virus. Exemplary virus proteins from non-enveloped viruses are from an adenovirus, astrovirus, circovirus, hepatitis E-like virus, papovavirus, parvovirus, picornavirus, or reovirus.

As noted above, the protein of interest may be a pathogen protein, wherein the pathogen is a bacterium. Exemplary bacterial proteins are selected from one of the bacteria Actinomyces israelii, Bacillus anthracis, Bacteroides fragilis, Bordetella pertussis, Borrelia, Brucella, Campylobacter jejuni, Chlamydia, Chlamydophila psittaci, Clostridium, Corynebacterium diphtheria, Ehrlichia, Enterococcus, Escherichia, Francisella tularensis, Haemophilus influenza, Helicobacter pylori, Klebsiella pneumonia, Legionella pneumophila, Leptospira species, Listeria monocytogenes, Mycobacterium, Mycoplasma pneumonia, Neisseria, Pseudomonas aeruginosa, Nocardia asteroids, Rickettsia rickettsia, Salmonella, Shigella, Staphylococcus, Streptococcus, Vibrio cholera, or Yersinia pestis. Similarly, the protein of interest may be a pathogen protein, wherein the pathogen is a fungus. Exemplary fungal proteins are selected from one of the fungi Candida, Aspergillus, Cryptococcus, Histoplasma, Pneumocystis, or Stachybotrys. As noted above, the protein of interest may be a pathogen protein, wherein the pathogen protein is a protozoan protein. Exemplary protozoan proteins are selected from one of Diplomodas, Parabasilia, Euglenozoa, Entamoebida, Stramenopila, Alveolata, Rhizaria, or Archaeplastida.

In another embodiment, the protein of interest is a biomarker. This is particularly advantageous as a patient may be diagnosed or monitored for a disease by analyzing a bodily fluid samples such as a serum sample. In case the biomarker is identified, the patient may be monitored more specifically. Preferred biomarkers, which may be proteins of interest are a tumorigenic biomarker or metabolic biomarker, more preferably wherein the biomarker is selected from a list consisting of S100 calcium-binding protein B, MIA protein (melanoma inhibitory activity), Prostate-specific antigen (PSA), human chorionic gonadotropin (hCG), Her2neu, Plastin-3, EGFR, carcinoembryonic antigen (CEA), cancer antigen 125 (CA 125), Carbohydrate antigen 19-9 (CA 19-9), Carbohydrate antigen 15-3 (CA 15-3), alpha-fetoprotein (AFP), Microfibril Associated Protein 4 (MFAP4), or ApoA-1. In particular, Her2neu, Plastin-3 and/or EGFR can be biomarkers for circulating tumor cells (CTCs).

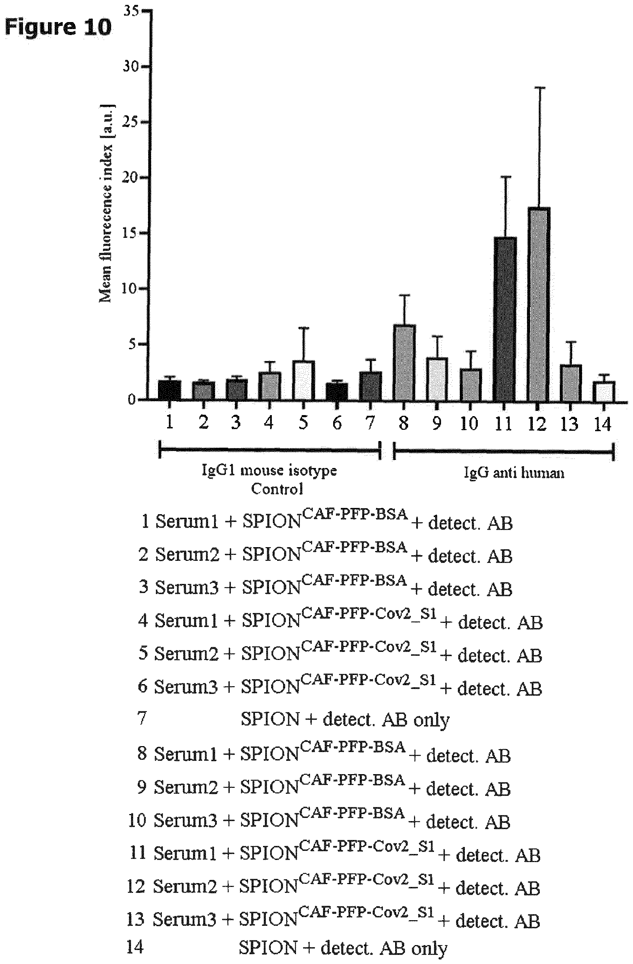

In a particularly preferred embodiment, the protein of interest is an antibody. As known in the art, are proteins produced in response to and counteracting a specific antigen. This may, for example, occur in response to a vaccination. In particular, antibodies against Sars-CoV-2 may be formed in response to a vaccination. Especially, antibodies against the spike protein of SarS-CoV 2 may be formed in response to a vaccination. Alternatively, the antibodies may be formed in response to a natural infection. Furthermore, the antibody status allows physicians and scientist to monitor the antibody positive and negative rate in the population over time. Hence, the method as disclosed in this application is useful in assessing e.g. the antibody status against SarS-Cov2 as demonstrated in Example 1, **Fig.** 10. As the linkage between the iron-based particles and the bait molecule merely requires the bait molecule to have an amino group or a thiol group, said linking mechanism is widely applicable. In a preferred embodiment, the protein of interest is a neutralizing antibody.

In a preferred embodiment, the protein of interest is an antibody having been formed in response to an infection by a pathogen or a vaccination against said pathogen. For example, antibodies against Sars-CoV2 are formed in response to an infection or vaccination. In general, said antibodies may have been formed in response to a viral or bacterial pathogen. Other examples of a possible pathogens, against which antibodies may have been formed, include Sars-CoV2, corynebacterium diphtheriae, haemophilus influenzae type b, hepatitis B virus, measles virus, meningococcus (Neisseria meningitidis), mumps virus, bordetella pertussis, pneumococcus (Streptococcus pneumoniae), poliovirus, rotavirus, rubella virus, Clostridium tetani, varicella zoster virus, human papillomavirus, herpes zoster, and influenza virus. Further example of pathogens, against which antibodies may have been formed, may be enveloped or a non-enveloped viruses, preferably an enveloped virus. For example, the enveloped virus, against which antibodies may have been formed, may be a coronavirus, retrovirus, herpesvirus, poxvirus, hepadnavirus, african swine fever and related viruses, flavivirus, alphavirus, togavirus, hepatitis D virus, orthomyxovirus, paramyxovirus, rhabdovirus, bunyavirus, or filovirus. Specifically, said enveloped virus may be coronavirus, preferably wherein the coronavirus is a orthocoronavirus, preferably a betacoronavirus, more preferably a sarbecovirus, even more preferably a SARS-coronavirus, and most preferably SARS-coronavirus 2.

In another preferred embodiment, the protein of interest is an auto-antibody. The skilled person is aware of auto-antibodies, which generally form in the context of an autoimmune disease. Specifically, the auto-antibody may be selected from a list consisting of antinuclear antibodies, anti-transglutaminase antibodies, anti-ganglioside antibodies, anti-thrombin antibodies, anti-thyroid autoantibodies, anti-myelin basic protein, anti-myelin oligodendrocyte glycoprotein, anti-citrullinated protein, anti-rheumatoid factor, antimicrosomal antibodies, anti-thyroid plasma membrane antibodies, IgA endomysium antibodies, IgA tissue transglutaminase antibodies, anti-Human Cardiac Myosin antibodies, anti-thyroid peroxidase antibodies, anti-thyroglobulin antibodies, anti-Saccharomyces cerevisiae antibodies, anti-flagellin (CBir1), anti-neutrophil cytoplasmic antibodies, anti-glutamic acid decarboxylase antibodies, thyroid receptor autoantibodies (TRAK), anti-acetylcholine receptor, anti-double-stranded DNA antibodies, anti-Sm, anti-histone antibodies, anti-type IV collagen antibodies, anti-Scl-70 antibodies, anti-centromere antibodies, Anti-Jo-1 antibodies, anti-Proteinase 3 antibodies, anti-Plakoglobin antibodies, anti-Desmoglein III antibodies, and anti-Sjögren's-syndrome-related antigen A autoantibodies.

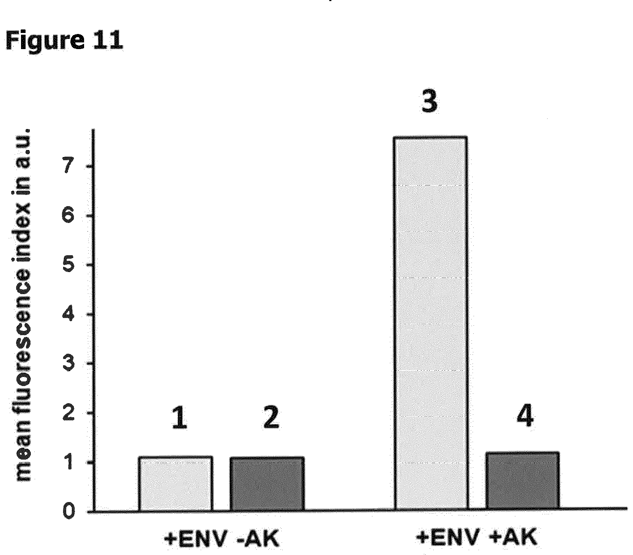

The term 'bait molecule', as used herein, is a macromolecule, which is used to catch a protein of interest. As noted above, the protein of interest and the bait protein have a high affinity for each other such as nanomolar affinity. Hence, when the bait molecule is contacted with the sample containing the protein of interest so that the protein of interest binds to the bait protein. It is preferred that the bait molecule is a protein. For example, the bait protein may be a pathogen protein, preferably the bait protein may a viral, bacterial, fungal or protozoan pathogen protein. In other words, the bait protein may be from a viral, bacterial, fungal or protozoan pathogen. For example, **Example 1** and **2** demonstrates that the pathogen proteins Sars-CoV-2 (spike protein) or HIV (GP120) may be used as bait proteins. Other possible bait proteins include viral protein from enveloped or non-enveloped viruses. In case the virus is an enveloped virus, the virus may be selected from a list consisting of coronavirus, retrovirus, herpesvirus, poxvirus, hepadnavirus, african swine fever and related viruses, flavivirus, alphavirus, togavirus, hepatitis D virus, orthomyxovirus, paramyxovirus, rhabdovirus, bunyavirus, or filovirus. Specifically, the enveloped virus may be a coronavirus, preferably wherein the coronavirus is a orthocoronavirus, preferably a betacoronavirus, more preferably a sarbecovirus, even more preferably a SARS-coronavirus or a MERS-coronavirus, even more preferably a SARS coronavirus, and most preferably SARS-coronavirus 2. Particular reference is made to Example 1, **Figure 10****,** showing that the spike protein of SARS-CoV2 can be used as a bait protein to identify Sars-CoV-2 antibodies in serum, i.e. antibodies against the spike protein. Alternatively, the bait protein may be from a retrovirus such as an orthoretrovirus, preferably wherein the retrovirus may be a lentivirus, more preferably wherein the retrovirus is human immunodeficiency viruses (HIV). Particular reference is made to **Example 2,** **Figure 11****,** showing that the envelope protein GP120 of HIV can be used as a bait protein to identify anti-HIV antibodies in solution, i.e. antibodies against the GP120. Alternatively, the bait protein may be from a non-enveloped virus such as an adenovirus, astrovirus, circovirus, hepatitis E-like virus, papovavirus, parvovirus, picornavirus, or reovirus. In addition the bait protein may be a bacterial protein from a bacterial pathogen, such as a bacterial protein is selected from one of the bacteria Actinomyces israelii, Bacillus anthracis, Bacteroides fragilis, Bordetella pertussis, Borrelia, Brucella, Campylobacter jejuni, Chlamydia, Chlamydophila psittaci, Clostridium, Corynebacterium diphtheria, Ehrlichia, Enterococcus, Escherichia, Francisella tularensis, Haemophilus influenza, Helicobacter pylori, Klebsiella pneumonia, Legionella pneumophila, Leptospira species, Listeria monocytogenes, Mycobacterium, Mycoplasma pneumonia, Neisseria, Pseudomonas aeruginosa, Nocardia asteroids, Rickettsia rickettsia, Salmonella, Shigella, Staphylococcus, Streptococcus, Vibrio cholera, or Yersinia pestis. Similarly, the bait protein may be a fungal protein, preferably wherein the fungal protein is selected from one of the fungi Candida, Aspergillus, Cryptococcus, Histoplasma, Pneumocystis, or Stachybotrys. Additionally, the bait protein may be a protozoan protein, preferably wherein the protozoan protein is from Diplomodas, Parabasilia, Euglenozoa, Entamoebida, Stramenopila, Alveolata, Rhizaria, or Archaeplastida.

It is also contemplated that the bait protein is an antibody, especially an antibody binding to a protein of interest as defined in the embodiments concerning the protein of interest in this application. Alternatively, the bait molecule may bind to a tag on the protein of interest. Exemplary tags include a Strep-Tag, SNAP-Tag, Clig-Tag, His-Tag, aldehyde Tag, FLAG-Tag, Flash-Tag, Thioreduxin-Tag or wherein the bait protein comprises protein A.

Furthermore, the bait molecule can be a bait aptamere. As known in the art, an aptamere is short, single-stranded DNA or RNA (ssDNA or ssRNA) molecule that can selectively bind to a specific target, including proteins, peptides, carbohydrates, small molecules, and toxins. Aptamer are capable of targeting a range from small organic molecules, ions, small peptides [11,12], large proteins [13-17], and whole cells and viruses. Aptamers usually show affinities in the pM to low nM range. Protein targets include viral proteins, cytokines, enzymes, and transcription factors. In the context of this application, a 'bait aptamere' bind to the protein of interest. Preferably, the bait aptamere bind to the protein of interest with an affinity lower than 100 nM. The skilled person is aware of suitable methods to detect the affinity of an aptamere-protein interaction such as change of the refractive index in surface plasmon resonance (SPR) by e.g. Cytiva Biacore 8K; Surface plasmon resonance imaging (SPRi) e.g. by Horiba XelPlex; or Bio-Layer Interferometry e.g. by an Octet or Octet Red (ForteBio) in comparison to a reference sample. Furthermore, aptameres which can be used as bait aptameres are commercially available. In addition, there are selective methods such as SELEX to engineer aptamers very efficiently and with high binding constants [22, 23]. Particularly preferred aptameres are bait aptameres binding to any of the proteins of interest as defined in this application. Exemplary bait aptameres bind to VEGF, Sars-CoV2 or cocaine or the exemplary bait aptamere is Pegaptanib or BC 007.

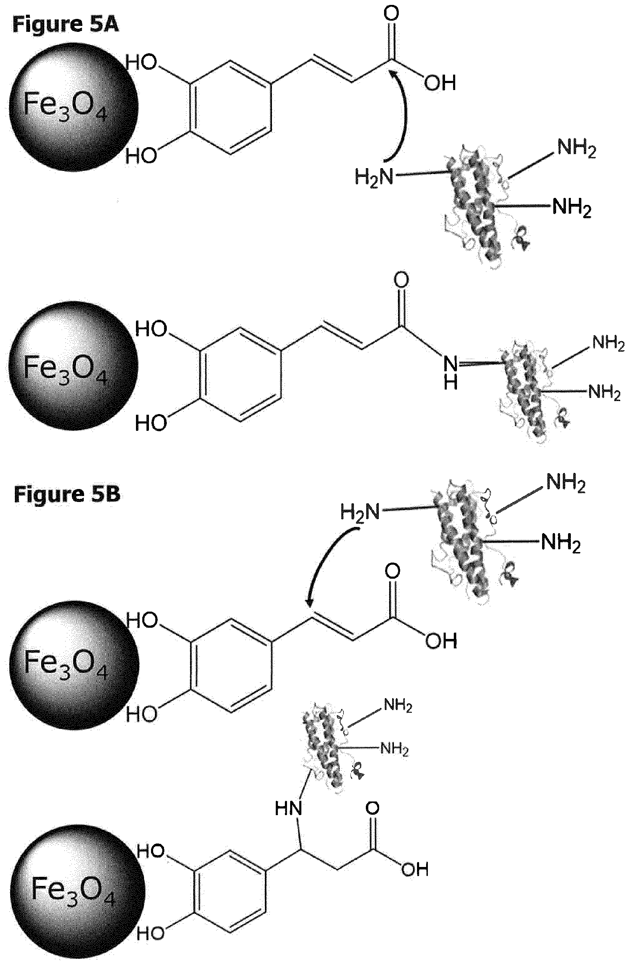

It is further contemplated that the amino group of the bait molecule is an N-terminal amino group, an amino group of a lysine, or an amino group of an arginine. In general, the amino group may be covalently linked to the catechol acids via an amid synthesis or a Michael reaction. Specifically, the reaction can occur following the principles of an amid synthesis as known in the art (see also **Fig. 5A** for illustration). Briefly, the acid group, such as the carboxylic acid group of the catechol acid is coupled with an amine, specifically an amino group of the bait molecule. For a direct reaction, e.g. between a carboxyl group and an amino group to form an amide, high temperatures are generally required. Hence, the catechol acid is activated by converting it to a better electrophile, as detailed in this disclosure. Alternatively, the bait molecule might be covalently linked to the activated catechol acid via a thiol group. For example, the thiol group may be part of a cysteine. In this case, the bait molecule is a bait protein harboring one or more cysteines.

The amino group or the thiol group may be reacting with the activated catechol acid via a Michael reaction **(****Fig.** 5B). In general, the Michael reaction, also called Michael addition is the nucleophilic addition of a nucleophile to an α,β-unsaturated carbonyl compound containing an electron withdrawing group and the skilled person is aware of the Michael reaction.

The "sample", as used herein is an aqueous of body fluid sample. In general, an "aqueous solution" is a solution in which the solvent is water. In other words, an aqueous solution is water soluble and the skilled person is aware of aqueous solutions. Exemplary aqueous solutions are waste water, ground water, or drinking water. Furthermore, a "body fluid" is any fluid, which occurs in or is excrete from a body. Especially preferred are samples from a human body. In particular, the sample may be a body fluid sample, which may be selected from blood, serum, plasma, lymph, urine, liquor, saliva and sputum, even more preferably wherein the human body fluid is blood or serum, and even more preferably wherein the human body fluid is blood. If the sample is blood of a body, it is preferred that the blood sample is a mammalian blood sample, more preferably a primate blood sample, and even more preferably a human blood sample. If the sample is serum of a body, it is preferred that the serum sample is a mammalian serum sample, more preferably a primate serum sample, and even more preferably a human serum sample. Additionally, the mammalian blood or serum samples may be from a group selected from cow, pig, rabbit, sheep, goat, mouse, rat, cat, dog, mouse, and rat. Advantageously, the method as disclosed herein can also be used on samples from farm animals as well as pets.

In general, "superparamagnetic iron-based particles" as used herein are particles, which are magnetically attractable. The skilled person is aware that the magnetic behaviour of materials can be classified into five major groups: ferro-, ferri-, para-, antiferro-, and diamagnetism. Ferro- and ferrimagnetism are the basic mechanisms in permanent magnets. Permanent magnets can be magnetized by external magnetic fields and remain magnetized after the removal of the fields. Materials such as Fe (iron), Co, Ni, and their alloys, and some compounds of rare earth elements are ferromagnetic. Superparamagnetism is a form of magnetism, which appears in small ferromagnetic or ferrimagnetic particles. "Particles" as used herein are defined as particles of matter that are from 1 nm (nanometer) to 2 micrometers (µm) in diameter. Furthermore, the skilled person is aware of iron (Fe) based particles, such as ironoxide based particles, which have been used in biological applications. The skilled person is also aware of various iron-based particles. For example, the particles may be iron oxide, iron (Fe), iron-cobalt, alnico or permalloy particles. In other words, the superparamagnetic iron-based particles may be selected from iron oxide, iron (Fe), ironcobalt, alnico, and permalloy particles. In a preferred embodiment, the superparamagnetic iron-based particles are superparamagnetic ironoxide nanoparticles (SPIONs). A "nanoparticle", as used herein, is a particle of matter that is between 1 nm and 1 µm in diameter. Furthermore, the superparamagnetic iron-based particles may be superparamagnetic ironoxide nanoparticles (SPIONs), more preferably wherein the iron oxide is selected from Fe₃O₄, γ-Fe₂O₃, CoFe₂O₄ or a mixture thereof, more preferably wherein the iron oxide is Fe₃O₄. In a preferred embodiment, the concentration of the superparamagnetic iron-based particles is determined by atomic emission spectroscopy (AES). The iron content in a solution may, for example, be determined with Atomic Emission Spectroscopy (AES). The skilled person is also aware of other methods in order to determine the iron content in a sample. For example, the concentration of iron in a sample can also be determined photometrically. However, a determination of the iron concentration by AES is preferred.

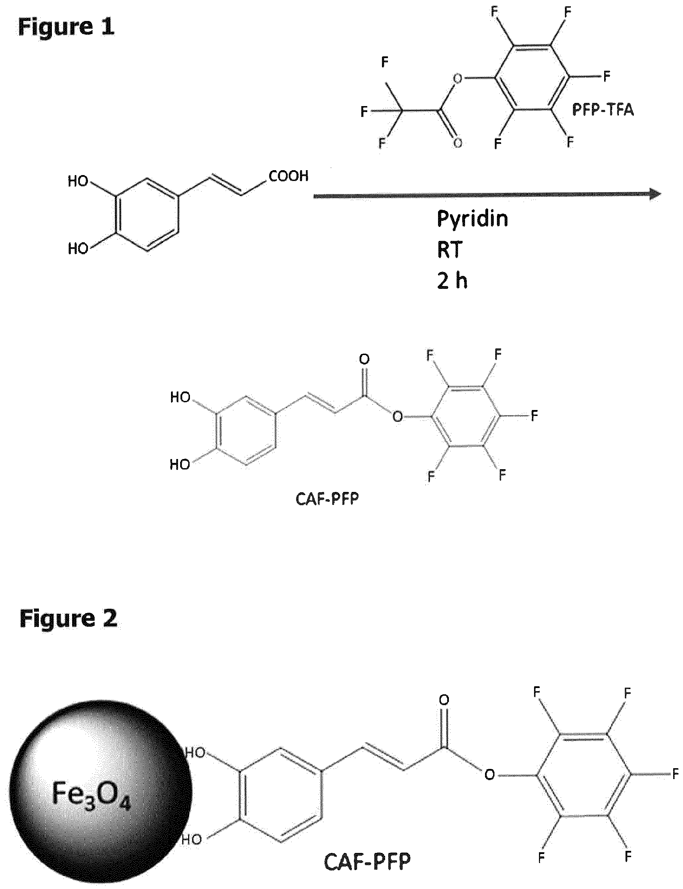

According to this disclosure, B has been bound to A (the superparamagnetic iron-based particle). Preferably, the catechol acid moiety (B) has been bound to the superparamagnetic iron-based particle (A) by a ligand exchange reaction. For example, this ligand exchange reaction may be performed with superparamagnetic iron-based particles previously coated with citrate **(****Fig.** 2). Further reference is made to **Example 1** herein. Preferably, the superparamagnetic iron-based particles are linked to the catechol acid via the citrate. In a particularly preferred embodiment, the superparamagnetic iron-based particles are coated with citrate following the protocol as described in Stein et al. Molecules 2020, 25(19) [8]. Alternatively, the superparamagnetic iron-based particles may be coated with citrate, oxalic acid, ascorbic acid, amino acids, preferably citrate. The advantage of a ligand exchange reaction is that it is easily applicable as shown in **Example 1** herein.

Alternatively, B has been bound to A directly *in situ.* For example, an alkaline precipitation of the naive raw superparamagnetic iron-based particles may be performed so that the activated catechol acid may directly attach to the superparamagnetic iron-based particles. Furthermore, B has been bound to A and said bond has been described in the art such as in Zeiniger et al. [20].

According to step (b) of the method, the complex may contacted with the sample in step b) for at least 5 minutes, preferably at least 10 minutes, more preferably at least 20 minutes, more preferably at least 30 minutes, even more preferably at least 45 minutes, even more preferably at least 1 hour, even more preferably at least 1.5 hours, and most preferably for about 2 hours. In a preferred embodiment, the complex is contacted for at most 24 hour, more preferably at most 18 hours, more preferably at most 14 hours, more preferably at most 12 hours, more preferably at most 10 hours, even more preferably at most 8 hours, even more preferably at most 6 hours, and even more preferably at most 4 hours.

In step (c) of the method, the complex binding to the protein of interest of step b) is immobilized by a magnetic field. "Immobilization", as used herein, means that something is made immobile. In the present case, immobilization occurs by the influence of a magnetic field. This means that the magnetically attractable particles are immobilised by the magnet field. A suitable magnetic field can be determined by the skilled person. For example, a suitable magnetic field is capable of immobilizing the superparamagnetic iron based particles. Immobilization can be assessed e.g. by assessing the iron concentration in the supernatant sample. Exemplary magnetic field strengths applied in step (c) may be from 50-2000 mT, preferably wherein the magnetic field is from 80 to 1500 mT, more preferably wherein the magnetic field is from 100 mT to 1000 mT, even more preferably wherein the magnetic field is from 150 mT to 500 mT, even more preferably wherein the magnetic field is from 180 mT to 300 mT and most preferably wherein the magnetic field is around 210 mT. Immobilization of the superparamagnetic iron-based particles increases with increasing time periods. In light of the present disclosure, and in particular Example 1 and 2, the skilled person is able to determine suitable time periods for immobilization of the superparamagnetic iron-based particles. In a preferred embodiment, the superparamagnetic iron-based particles are immobilised in step (c) for a time period of at least 0.5 seconds, preferably at least 1 second, more preferably at least 2 seconds, even more preferably at least 3 seconds, more preferably at least 10 seconds, more preferably at least 20 seconds, more preferably at least 30 seconds, more preferably at least 45 seconds, more preferably at least 1 minute, even more preferably at least 2 minutes, most preferably at least 3 minutes preferably at least 5 minutes, more preferably at least 10 minutes, even more preferably at least 20 minutes, even more preferably at least 30 minutes, even more preferably at least 1 hour. It is also preferred that the superparamagnetic iron-based particles are immobilised in step (c) for a time period of at most 180 minutes, preferably at most 120 minutes, more preferably at most 90 minutes, more preferably at most 60 minutes, and even more preferably at most 30 minutes. Of course, the skilled person can freely combine the preferred time ranges for immobilization as disclosed herein. In a strongly preferred embodiment, the superparamagnetic iron-based particles are immobilised in step (c) for a time period from 15 seconds to 90 minutes, preferably from 30 seconds to 60 minutes, and more preferably from 1 minute to 10 minutes.

According to step (d) of the method as disclosed herein, the complex binding to the protein of interest of step c) is contacted with a detection molecule. Such a detection molecule may be a protein, such as an antibody, or an aptamere. In a preferred embodiment, the detection molecule is a protein, preferably an antibody, nanobody or camelid single domain antibody, preferably wherein the detection molecule is an antibody. Alternatively, the detection molecule may be an aptamere. Preferably, the detection molecule comprises a detectable label, preferably wherein the detection molecule is covalently linked to a detectable label. The term 'label' as used herein refers to a molecule, which may be made detectable. Preferred are labels, which can be detected by e.g. light emission upon light excitation such as fluorescent labels. The skilled person is aware of such suitable labels in the art. For example, the detectable label may be, preferably is, a fluorophore or a quantum dot, even more preferably wherein the detectable label is a fluorophore. In a particularly preferred embodiment, the detection molecule is a fluorescently or quantumdot labelled molecule and even more preferably wherein the detectable label is a fluorescently labelled molecule. Fluorescently labelled molecules, such as fluorescently labelled antibodies, are known to the skilled person and are also commercially available. In the context of the present invention, the detection molecule needs to be specific to an antigen of the protein of interest. Thus, particularly preferred are fluorescently labelled antibodies as detection molecules. Alternatively, the detection molecule may be detectable by a labelling molecule, preferably wherein the labelling molecule is linked to a detectable label, more preferably wherein the detectable label is a fluorophore or a quantum dot, even more preferably wherein the detectable label is a fluorophore, and most preferably wherein the labelling molecule is a fluorescently or quantum dot labelled molecule. The labelling molecule may be a protein or an aptamer. In case the labelling molecule is a protein, the labelling molecule is preferably an antibody, nanobody or camelid single domain antibody. It is even more preferred that the labelling molecule is an antibody.

In an especially preferred embodiment, the labelling molecule is secondary antibody which is fluorescently labelled, binding to a primary antibody. In practice, the skilled person is aware of typical detection and labelling molecules such as primary and secondary antibodies, as standardly used in mass spectrometry. Briefly, the detection molecule is directed against an antigen on the protein of interest, and the labelling molecule is directed against the detection molecule. Typical pairs of detection and labelling molecules are primary and secondary antibodies, wherein the secondary antibody specifically binds to the constant region of the primary antibody, which is specific to a species. For example, for a human sample, a primary antibody raised in mouse may be used and a secondary antibody against the mouse constant region raised in rabbit may be used.

According to the present disclosure, the identity of the protein of interest is determined by the presence of the label. Ideally, the presence of the label is measured by a cell analyser, preferably by flow cytometry or by microcapillary cytometry, more preferably by flow cytometry. The skilled person is aware of standard cell analyser methods, which allow the detection of a label. Ideally, the label is present if the signal of the label is at least 2-fold higher than the negative control, preferably 3-fold, more preferably 4-fold, even more preferably 5-fold, even more preferably 6-fold, even more preferably 7-fold, even more preferably 8-fold, even more preferably 9-fold, even more preferably 10-fold, even more preferably 12-fold, even more preferably 15-fold, even more preferably 20-fold, and at most 1000-fold.

It is further contemplated that the complex of step a) has been obtained by following the steps of:
i. activating the catechol acid (B; benzene-1,2-diol acid);
ii. linking the activated catechol acid (B) to the superparamagnetic iron-based particle (A), and
iii. reacting said activated catechol acid with an amino group of the bait molecule (C).

It is further contemplated that after step (ii), the activating group of the catechol acid is cleaved and the catechol acid is subsequently reacted with the bait molecule. Said order of the reaction has the advantage of controlling the steps of the reaction, since the pentafluorophenol can be readily detected by HPLC-UV.

When carrying out the method as disclosed herein, it is preferred that 5-20 times of the iron weight amount of A is linked to one (1) weight amount of the bait molecule, preferably 7-15 times, more preferably, 8-13 times, even more preferably 9-11 times, and most preferably around 10 times. The iron weight amount can be determined by standard methods such as AES. Furthermore, the weight amount of the bait molecule can also be determined by standard methods. For example, the bait molecule may be a bait protein and the concentration may be determined by UV absorbance, e.g. via NanoDrop, or Bradford Assay.

It is even more preferred that 10-1000 ug iron of A is linked to 1-100 ug of the bait molecule (C), preferably wherein 20-500 ug iron of A is linked to 2-50 ug of C, more preferably wherein 40-200 ug iron of A is linked to 4-20 ug of C, even more preferably wherein 60-160 ug iron of A is linked to 6-16 ug of C, even more preferably wherein 80-130 ug iron of A is linked to 8-13 ug of C, and most preferably wherein around 100 ug iron of A is linked to around 10 ug of C.

Additionally, it is preferred that the complex comprises, in particular consists of, (A) a (one) superparamagnetic iron-based particle, (B) one or more catechol acid moieties, and (C) one or more bait molecules, wherein the number of bait molecules is not greater than the number of catechol acid moieties. This means that each catechol acid moiety (B) has been covalently linked to at most one bait molecule (C). In particular, catechol acid moieties do not form a sequential chain of catechol acid moieties to which the bait molecule is covalently linked. It is even more preferred that the number of bait molecules is equal to the number of catechol acid moieties. This means that each catechol acid moiety (B) has been covalently linked to one bait molecule (C) forming a complex of the formula: A-[B-C]ₙ, wherein n is at least 1, more preferably at most 200.

It is also preferred that the complex consists of a superparamagnetic iron-based particle (A), one or more catechol acid moieties (B), and one or more and bait molecules (C). In practice, numerous complexes are required for the identification of numerous proteins of interest as not a single protein of interest can be detected in practice. Based on the molecular weight of the bait molecule more or less bait molecules may be bound to the particles. The examples section as well as the preferred embodiments above provide guidance with regard to suitable amounts for A, B and C.

As quality control measure, the amino group or thiol group of bovine serum albumin (BSA) may be covalently bound to the complex of step (b) instead of the bait molecule and may thereby serve a negative control. **Example 1** shows that such a negative control is useful to in order to determine the background noise of the detection method.

In a further aspect of the invention, a complex comprising, preferably consisting of
(A) a superparamagnetic iron-based particle,
(B) one or more catechol acid moieties, and
(C) one or more bait molecules,
wherein B has been bound to A; and wherein B has been covalently linked to C by activating the catechol acid (benzene-1,2-diol acid) and reacting said activated catechol acid with an amino group or a thiol group of C, is contemplated.

The preferred embodiments regarding the bait molecule, the superparamagnetic iron-based particles, the catechol acid, and/or the amino group of thiol group of the protein of interest are discussed in this disclosure and apply equally to this aspect of the invention.

Additionally, a protein of interest may be bound to the bait molecule (C). In other words, the complex as disclosed in this aspect of the invention may comprise, preferably consist of A, one or more of B, one or more of C and additionally one or more of protein of interest (D). Preferred embodiments regarding the protein of interest are further defined in this disclosure and apply equally to this aspect. In particular, the protein of interest may be an antibody and/or the bait molecule may be an antibody. In a very preferred embodiment, the complex consists of a superparamagnetic iron-based particle (A), one or more catechol acid moieties (B), and one or more bait molecules (C). For example, B may have been bound to A by a ligand exchange reaction. Preferred ligand exchange reactions are discussed in this disclosure and apply equally to this aspect of the invention. Alternatively, B may have been bound to A *in situ* by alkaline precipitation, which are also discussed in this disclosure and which applies equally to this aspect of the disclosure. Specifically, A may have been linked to a plurality of catechol acid moieties (B). Additionally, the plurality of catechol acid moieties (B) may have been linked to a plurality of bait molecules (C). The skilled person understands that one superparamagnetic iron-based particle (A) may be bound to one or more catechol acid moieties (B) in practice as several, i.e. a plurality of B can be bound to A. In turn, the skilled person also understands that one bait molecule (C) can be covalently bound to one catechol acid moiety (B). Hence, one superparamagnetic iron-based particle (A), may be bound to one or more catechol acid moieties (B), and one or more bait molecules (C), wherein the number of B and C ideally the same.

Furthermore, it is preferred that the complex comprises, in particular consists of, (A) a (one) superparamagnetic iron-based particle, (B) one or more catechol acid moieties, and (C) one or more bait molecules, wherein the number of bait molecules is not greater than the number of catechol acid moieties. This means that each catechol acid moiety (B) has been covalently linked to at most one bait molecule (C). In particular, catechol acid moieties do not form a sequential chain of catechol acid moieties to which the bait molecule is covalently linked. It is even more preferred that the number of bait molecules is equal to the number of catechol acid moieties. This means that each catechol acid moiety (B) has been covalently linked to one bait molecule (C) forming a complex of the formula: A-[B-C]ₙ, wherein n is at least 1, more preferably at most 200.

Additionally, a method for diagnosing a disease or disorder, wherein a protein of interest is identified in a body fluid sample of a patient is disclosed herein, wherein the method comprising the steps of:
a) providing a complex comprising, preferably consisting of,
   (A) a superparamagnetic iron-based particle,
   (B) one or more catechol acid moieties, and
   (C) one or more bait molecules,
      wherein B has been bound to A; and wherein B has been covalently linked to C by activating the catechol acid (benzene-1,2-diol acid) and reacting said activated catechol acid with amino groups of C;
b) contacting the complex of step a) with the sample whereby the bait molecule (C) binds to the protein of interest;
c) immobilizing the complex binding to the protein of interest of step b) by a magnetic field and thereby separating the protein of interest from the sample,
d) contacting the complex binding to the protein of interest of step c) with a detection molecule, wherein the detection molecule binds to the protein of interest; wherein the detection molecule (i) comprises a detectable label or (ii) is detectable by a labelling molecule comprising the label,
e) determining the presence of the label, thereby identifying the protein of interest.

Preferably, the presence of the protein of interest indicates that the patient has a risk of developing or suffers from said disease or disorder. Exemplary diseases or disorders are infectious diseases or auto-immune disorders, preferably wherein the disease or disorder is an infectious disease. Preferred infectious diseases are caused by a virus, bacterium, fungus or protozoan. In addition, preferred viruses bacteria, fungi or protozoans may be selected from a pathogen as defined in this disclosure. Moreover, preferred embodiments regarding the protein of interest, the sample, the superparamagnetic iron-based, the catechol acid, the amino group or thiol group of the protein of interest, and/or the bait molecule are defined in this disclosure and apply equally to this aspect of the invention.

In a further aspect, a method of preparing a complex is contemplated, wherein the method comprising the steps of:
I. providing a superparamagnetic iron-based particle (A),
II. activating catechol acid (B; benzene-1,2-diol acid);
III. linking the activated catechol acid (B) to A, and
IV. reacting said activated catechol acid with an amino group or thiol group of a bait molecule (C).

Preferred embodiments regarding the superparamagnetic iron-based particles, the catechol acid, and/or the bait molecule are defined in this disclosed and apply equally to this aspect of the invention.

Furthermore, a use of a complex for identifying a protein of interest in an aqueous or body fluid sample is disclosed herein, wherein a comprises, preferably consists of,
(A) a superparamagnetic iron-based particle,
(B) one or more catechol acid moieties, and
(C) one or more bait molecules,

wherein B has been bound to A; and wherein B has been covalently linked to C by activating the catechol acid (benzene-1,2-diol acid) and reacting said activated catechol acid with an amino group or thiol group of C;
and wherein the protein of interest is capable of binding to the bait molecule.

Preferred embodiments regarding the protein of interest, the aqueous or body fluid sample, the superparamagnetic iron-based particles, the catechol acid, the amino group or thiol group of the protein of interest, and/or the bait molecule, are defined in this disclosed and apply equally to this aspect of the invention.

Furthermore, it is preferred that the complex comprises, in particular consists of, (A) a (one) superparamagnetic iron-based particle, (B) one or more catechol acid moieties, and (C) one or more bait molecules, wherein the number of bait molecules is not greater than the number of catechol acid moieties. This means that each catechol acid moiety (B) has been covalently linked to at most one bait molecule (C). In particular, catechol acid moieties do not form a sequential chain of catechol acid moieties to which the bait molecule is covalently linked. It is even more preferred that the number of bait molecules is equal to the number of catechol acid moieties. This means that each catechol acid moiety (B) has been covalently linked to one bait molecule (C) forming a complex of the formula: A-[B-C]ₙ, wherein n is at least 1, more preferably at most 200.

Moreover, a kit for identifying a protein of interest in an aqueous or body fluid sample is contemplated, wherein the kit comprises a complex comprising, preferably consisting of:
(A) a superparamagnetic iron-based particle,
(B) one or more catechol acid moieties, and
(C) one or more bait molecules,

wherein B has been bound to A; and wherein B has been covalently linked to C by activating the catechol acid (benzene-1,2-diol acid) and reacting said activated catechol acid with amino groups of C;
and wherein the protein of interest is capable of binding to the bait molecule

Preferred embodiments regarding the protein of interest, the aqueous or body fluid sample, the superparamagnetic iron-based particles, the catechol acid, the amino group or thiol group of the protein of interest, and/or the bait molecule, are defined in this disclosed and apply equally to this aspect of the invention.

Furthermore, it is preferred that the complex comprises, in particular consists of, (A) a (one) superparamagnetic iron-based particle, (B) one or more catechol acid moieties, and (C) one or more bait molecules, wherein the number of bait molecules is not greater than the number of catechol acid moieties. This means that each catechol acid moiety (B) has been covalently linked to at most one bait molecule (C). In particular, catechol acid moieties do not form a sequential chain of catechol acid moieties to which the bait molecule is covalently linked. It is even more preferred that the number of bait molecules is equal to the number of catechol acid moieties. This means that each catechol acid moiety (B) has been covalently linked to one bait molecule (C) forming a complex of the formula: A-[B-C]ₙ, wherein n is at least 1, more preferably at most 200.

The invention is further described by the following embodiments:
1. A method for identifying a protein of interest in an aqueous or body fluid sample, the method comprising the steps of:
   a) providing a complex comprising
      (A) a superparamagnetic iron-based particle,
      (B) one or more catechol acid moieties, and
      (C) one or more bait molecules,
      wherein B has been bound to A; and wherein B has been covalently linked to C by activating the catechol acid (benzene-1,2-diol acid) and reacting said activated catechol acid with the amino group or the thiol group of C;
   b) contacting the complex of step a) with the sample whereby the bait molecule (C) binds to the protein of interest;
   c) immobilizing the complex binding to the protein of interest of step b) by a magnetic field and thereby separating the protein of interest from the sample,
   d) contacting the complex binding to the protein of interest of step c) with a detection molecule, wherein the detection molecule binds to the protein of interest; wherein the detection molecule (i) comprises a detectable label or (ii) is detectable by a labelling molecule comprising the label,
   e) determining the presence of the label,
      thereby identifying the protein of interest.
2. The method of embodiment 1, wherein the protein of interest is a pathogen protein, preferably wherein the pathogen protein is a viral protein, a bacterial protein, a fungal protein, or a protozoan protein.
3. The method of embodiment 2, wherein the viral protein is from an enveloped or a non-enveloped virus, preferably wherein the viral protein is from an enveloped virus.
4. The method of embodiment 3, wherein the enveloped virus is a coronavirus, retrovirus, herpesvirus, poxvirus, hepadnavirus, african swine fever and related viruses, flavivirus, alphavirus, togavirus, hepatitis D virus, orthomyxovirus, paramyxovirus, rhabdovirus, bunyavirus, or filovirus.
5. The method of embodiment 4, wherein the enveloped virus is a coronavirus, preferably wherein the coronavirus is a orthocoronavirus, preferably a betacoronavirus, more preferably a sarbecovirus, even more preferably a SARS-coronavirus or a MERS-coronavirus, even more preferably a SARS coronavirus, and most preferably SARS-coronavirus 2.
6. The method of embodiment 4, wherein the retrovirus is an orthoretrovirus, preferably wherein the retrovirus is a lentivirus, more preferable wherein the retrovirus is human immunodeficiency viruses (HIV).
7. The method of embodiment 3, wherein the non-enveloped virus is an adenovirus, astrovirus, circovirus, hepatitis E-like virus, papovavirus, parvovirus, picornavirus, or reovirus.
8. The method of embodiment 2, wherein the pathogen protein is a bacterial protein, preferably wherein the bacterial protein is selected from one of the bacteria Actinomyces israelii, Bacillus anthracis, Bacteroides fragilis, Bordetella pertussis, Borrelia, Brucella, Campylobacter jejuni, Chlamydia, Chlamydophila psittaci, Clostridium, Corynebacterium diphtheria, Ehrlichia, Enterococcus, Escherichia, Francisella tularensis, Haemophilus influenza, Helicobacter pylori, Klebsiella pneumonia, Legionella pneumophila, Leptospira species, Listeria monocytogenes, Mycobacterium, Mycoplasma pneumonia, Neisseria, Pseudomonas aeruginosa, Nocardia asteroids, Rickettsia rickettsia, Salmonella, Shigella, Staphylococcus, Streptococcus, Vibrio cholera, or Yersinia pestis.
9. The method of embodiment 2, wherein the pathogen protein is a fungal protein, preferably wherein the fungal protein is selected from one of the fungi Candida, Aspergillus, Cryptococcus, Histoplasma, Pneumocystis, or Stachybotrys.
10. The method of embodiment 2, wherein the pathogen protein is a protozoan protein, preferably wherein the protozoan protein is from Diplomodas, Parabasilia, Euglenozoa, Entamoebida, Stramenopila, Alveolata, Rhizaria, or Archaeplastida.
11. The method of embodiment 1, wherein the protein of interest is a biomarker, preferably a tumorigenic biomarker or metabolic biomarker, more preferably wherein the biomarker is selected from a list consisting of S100 calcium-binding protein B, MIA (melanoma inhibitory activity), Prostate-specific antigen (PSA), human chorionic gonadotropin (hCG), circulating tumor cells (CTCs), carcinoembryonic antigen (CEA), cancer antigen 125 (CA 125), Carbohydrate antigen 19-9 (CA 19-9), Carbohydrate antigen 15-3 (CA 15-3), alpha-fetoprotein (AFP), Microfibril Associated Protein 4 (MFAP4), and ApoA-1.
12. The method of embodiment 1, wherein the protein of interest is an antibody, preferably wherein the antibody is a neutralizing antibody.
13. The method of embodiment 12, wherein the protein of interest is an antibody and wherein said antibody binds to the bait molecule.
14. The method of any of embodiments 1, 12 and 13, wherein the protein of interest is an antibody having been formed in response to an infection by a pathogen or a vaccination against said pathogen.
15. The method of embodiment 14, wherein the pathogen is a viral or bacterial pathogen.
16. The method of embodiment 15, wherein the pathogen is selected from a list consisting of Sars-CoV2, corynebacterium diphtheriae, haemophilus influenzae type b, hepatitis B virus, measles virus, meningococcus (Neisseria meningitidis), mumps virus, bordetella pertussis, pneumococcus (Streptococcus pneumoniae), poliovirus, rotavirus, rubella virus, Clostridium tetani, varicella zoster virus, human papillomavirus, herpes zoster, and influenza virus.
17. The method of embodiment 15, wherein the pathogen is a viral pathogen, preferably wherein the viral pathogen is an enveloped or a non-enveloped virus, preferably wherein the viral pathogen is an enveloped virus.
18. The method of embodiment 17, wherein the enveloped virus is a coronavirus, retrovirus, herpesvirus, poxvirus, hepadnavirus, african swine fever and related viruses, flavivirus, alphavirus, togavirus, hepatitis D virus, orthomyxovirus, paramyxovirus, rhabdovirus, bunyavirus, or filovirus.
19. The method of embodiment 18, wherein the enveloped virus is a coronavirus, preferably wherein the coronavirus is a orthocoronavirus, preferably a betacoronavirus, more preferably a sarbecovirus, even more preferably a SARS-coronavirus, and most preferably SARS-coronavirus 2.
20. The method of any of embodiments 1, 12 and 13, wherein the protein of interest is an antibody, preferably wherein the protein of interest is an auto-antibody, more preferably wherein the auto-antibody is selected from a list consisting of antinuclear antibodies, anti-transglutaminase antibodies, anti-ganglioside antibodies, anti-thrombin antibodies, anti-thyroid autoantibodies, anti-myelin basic protein, anti-myelin oligodendrocyte glycoprotein, anti-citrullinated protein, anti-rheumatoid factor, antimicrosomal antibodies, anti-thyroid plasma membrane antibodies, IgA endomysium antibodies, IgA tissue transglutaminase antibodies, anti-Human Cardiac Myosin antibodies, anti-thyroid peroxidase antibodies, anti-thyroglobulin antibodies, anti-Saccharomyces cerevisiae antibodies, anti-flagellin (CBir1), anti-neutrophil cytoplasmic antibodies, anti-glutamic acid decarboxylase antibodies, thyroid receptor autoantibodies (TRAK), anti-acetylcholine receptor, anti-double-stranded DNA antibodies, anti-Sm, anti-histone antibodies, anti-type IV collagen antibodies, anti-Scl-70 antibodies, anti-centromere antibodies, anti-Jo-1 antibodies, anti-Proteinase 3 antibodies, anti-Plakoglobin antibodies, anti-Desmoglein III antibodies, and anti-Sjogren's-syndrome-related antigen A autoantibodies.
21. The method of any of the preceding embodiments, wherein the amino group of the bait molecule is an N-terminal amino group, an amino group of a lysine, or an amino group of an arginine; and/or the thiol group of the bait molecule is a thiol group of a cysteine.
22. The method of any of the preceding embodiments, wherein the bait molecule comprises a binding site for the protein of interest, preferably wherein the bait molecule provides an antigen for the protein of interest and wherein the protein of interest is an antibody.
23. The method of any of the preceding embodiments, wherein the bait molecule is a protein, preferably a pathogen protein, more preferably wherein the pathogen protein is a viral protein, a bacterial protein, a fungal protein, or a protozoan protein.
24. The method of embodiment 23, wherein the viral protein is from an enveloped or a non-enveloped virus, preferably wherein the viral protein is from an enveloped virus.
25. The method of embodiment 24, wherein the enveloped virus is a coronavirus, retrovirus, herpesvirus, poxvirus, hepadnavirus, african swine fever and related viruses, flavivirus, alphavirus, togavirus, hepatitis D virus, orthomyxovirus, paramyxovirus, rhabdovirus, bunyavirus, or filovirus.
26. The method of embodiment 25, wherein the enveloped virus is a coronavirus, preferably wherein the coronavirus is a orthocoronavirus, preferably a betacoronavirus, more preferably a sarbecovirus, even more preferably a SARS-coronavirus or a MERS-coronavirus, even more preferably a SARS coronavirus, and most preferably SARS-coronavirus 2 .
27. The method of embodiment 25, wherein the retrovirus is an orthoretrovirus, preferably wherein the retrovirus is a lentivirus, more preferable wherein the retrovirus is human immunodeficiency viruses (HIV).
28. The method of embodiment 24, wherein the non-enveloped virus is an adenovirus, astrovirus, circovirus, hepatitis E-like virus, papovavirus, parvovirus, picornavirus, or reovirus.
29. The method of embodiment 23, wherein the pathogen protein is a bacterial protein, preferably wherein the bacterial protein is selected from one of the bacteria Actinomyces israelii, Bacillus anthracis, Bacteroides fragilis, Bordetella pertussis, Borrelia, Brucella, Campylobacter jejuni, Chlamydia, Chlamydophila psittaci, Clostridium, Corynebacterium diphtheria, Ehrlichia, Enterococcus, Escherichia, Francisella tularensis, Haemophilus influenza, Helicobacter pylori, Klebsiella pneumonia, Legionella pneumophila, Leptospira species, Listeria monocytogenes, Mycobacterium, Mycoplasma pneumonia, Neisseria, Pseudomonas aeruginosa, Nocardia asteroids, Rickettsia rickettsia, Salmonella, Shigella, Staphylococcus, Streptococcus, Vibrio cholera, or Yersinia pestis.
30. The method of embodiment 23, wherein the pathogen protein is a fungal protein, preferably wherein the fungal protein is selected from one of the fungi Candida, Aspergillus, Cryptococcus, Histoplasma, Pneumocystis, or Stachybotrys.
31. The method of embodiment 23, wherein the pathogen protein is a protozoan protein, preferably wherein the protozoan protein is from Diplomodas, Parabasilia, Euglenozoa, Entamoebida, Stramenopila, Alveolata, Rhizaria, or Archaeplastida.
32. The method of any of the preceding embodiments, wherein the bait molecule is an antibody, preferably wherein the bait molecule is an antibody binding to a protein of interest as defined in any of embodiments 2-20.
33. The method of any of the preceding embodiments, wherein the bait molecule binds to a tag on the protein of interest, preferably wherein the tag is a Strep-Tag, SNAP-Tag, Clig-Tag, His-Tag, Aldehyde Tag, FLAG-Tag, Flash-Tag, Thioreduxin-Tag or wherein the bait protein comprises protein A.
34. The method of any of the preceding embodiments, wherein the bait molecule is a bait aptamere.
35. The method of embodiment 34, wherein the bait aptamere bind to the protein of interest with an affinity lower than 100 nM.
36. The method of embodiments 34 or 35, wherein the bait aptamere binds to the any of the protein of interest as defined in embodiments 2-20.
37. The method of any of embodiments 33-36, wherein the bait aptamere binds to VEGF, Sars-CoV2 or cocaine; or wherein the bait aptamere is Pegaptanib or BC 007.
38. The method of any of the preceding embodiments, wherein the aqueous or body fluid sample is a body fluid, waste water, ground water, or drinking water, preferably wherein the body fluid sample is a human body fluid, more preferably wherein the human body fluid is selected from blood, serum, plasma, lymph, urine, liquor, saliva and sputum, even more preferably wherein the human body fluid is blood or serum, and even more preferably wherein the human body fluid is blood.
39. The method of any of the preceding embodiments, wherein the sample is a blood sample, preferably wherein the blood sample is a mammalian blood sample, more preferably a primate blood sample, and even more preferably a human blood sample.
40. The method of any of the preceding embodiments, wherein the superparamagnetic iron-based particle (A) has been bound to catechol acid moieties (B) by a ligand exchange reaction.
41. The method of any of the preceding embodiments, wherein the superparamagnetic iron-based particles are selected from an iron oxide, iron (Fe), iron-cobalt, alnico, permalloy particles.
42. The method of any of the preceding embodiments, wherein the superparamagnetic iron-based particles are superparamagnetic ironoxide nanoparticles (SPIONs).
43. The method of any of the preceding embodiments, wherein the superparamagnetic iron-based particles are superparamagnetic ironoxide nanoparticles (SPIONs), more preferably wherein the iron oxide is selected from Fe₃O₄, γ-Fe₂O₃, CoFe₂O₄ or a mixture thereof, more preferably wherein the iron oxide is Fe₃O₄.
44. The method of any of the preceding embodiments, wherein the superparamagnetic iron-based particles are coated with citrate, oxalic acid, ascorbic acid, amino acids, preferably citrate.
45. The method of embodiment 44, wherein the superparamagnetic iron-based particles are coated with citrate following the protocol as described in Stein et al. Molecules 2020, 25(19).
46. The method of embodiments 44 or 45, wherein the superparamagnetic iron-based particles are linked to the catechol acid via the citrate.
47. The method of any of the preceding embodiments, wherein the concentration of the superparamagnetic iron-based particles is determined by atomic emission spectroscopy.
48. The method of any of the preceding embodiments, wherein the catechol acid comprises a carboxyl group, preferably wherein the catechol acid is caffeic acid (3,4-dihydroxycinnamic acid) or protocatechuic acid, more preferably wherein the catechol acid is caffeic acid.
49. The method of any of the preceding embodiments, wherein the catechol acid has been activated to be a pentafluorophenyl catechol acid, terafluorophenyl catechol acid, or a catechol acid N-Hydroxysuccinimide ester, preferably wherein the catechol acid has been activated to be a pentafluorophenyl catechol acid.
50. The method of any of the preceding embodiments, wherein the catechol acid has been activated with a isocyanate, acyl azides, a sulfonyl chloride, a tosylate ester, an epoxide, an oxiranes, an imido ester, a carbodiimide, a fluorophenyl ester, or a O-methyl isourea.
51. The method of any of the preceding embodiments, wherein the catechol acid is caffeic acid, preferably wherein the caffeic acid has been activated to be a pentafluorophenyl caffeic acid, terafluorophenyl caffeic acid, or a caffeic acid N-Hydroxysuccinimide ester.
52. The method of any of the preceding embodiments, wherein the catechol acid has been activated by pentafluorophenyl trifluoroacetate.
53. The method of any of the preceding embodiments, wherein the catechol acid is caffeic acid and wherein pentafluorophenol caffeic acid ester has been formed by the activation of caffeic acid.
54. The method of any of the preceding embodiments, wherein the catechol acid is caffeic acid and wherein the caffeic acid has been activated by pentafluorophenyl trifluoroacetate.
55. The method of any of the preceding embodiments, wherein the complex is contacted with the sample in step b) for at least 5 minutes, preferably at least 10 minutes, more preferably at least 20 minutes, more preferably at least 30 minutes, even more preferably at least 45 minutes, even more preferably at least 1 hour, even more preferably at least 1.5 hours, and most preferably for about 2 hours.
56. The method of any of the preceding embodiments, wherein the complex is contacted with the sample in step b) for at most 10 hours, preferably at most 8 hours, more preferably at most 6 hours, more preferably at most 5 hours, more preferably at most 4 hours, more preferably at most 3 hours, and most preferably about 2 hours.
57. The method of any of the preceding embodiments, wherein the magnetic field applied in step (c) is from 50-2000 mT, preferably wherein the magnetic field is from 80 to 1500 mT, more preferably wherein the magnetic field is from 100 mT to 1000 mT, even more preferably wherein the magnetic field is from 150 mT to 500 mT, even more preferably wherein the magnetic field is from 180 mT to 300 mT and most preferably wherein the magnetic field is around 210 mT.
58. The method of any of the preceding embodiments, wherein the complex of step (c) is contacted with the detection molecule for at least 1 minute, preferably at least 2 minutes, preferably at least 5 minutes, more preferably at least 10 minutes, even more preferably at least 20 minutes, even more preferably at least 30 minutes, even more preferably at least 1 hour, and most preferably for about 1 hour.
59. The method of any of the preceding embodiments, wherein the detection molecule is a protein or an aptamer.
60. The method of embodiment 59, wherein the detection molecule is a protein, preferably an antibody, a nanobody or a camelid single domain antibody, preferably wherein the detection molecule is an antibody.
61. The method of embodiment 59, wherein the detection molecule is an aptamere.
62. The method of any of the preceding embodiments, wherein the detection molecule comprises a detectable label, preferably wherein the detection molecule is covalently linked to a detectable label.
63. The method of embodiment 62, wherein the detectable label is a fluorophore or a quantum dot, even more preferably wherein the detectable label is a fluorophore, even more preferably wherein the detection molecule is a fluorescently or quantumdot labelled molecule and even more preferably wherein the detectable label is a fluorescently labelled molecule.
64. The method of any of embodiments 1-61, wherein the detection molecule is detectable by a labelling molecule, preferably wherein the labelling molecule is linked to a detectable label, more preferably wherein the detectable label is a fluorophore or a quantum dot, even more preferably wherein the detectable label is a fluorophore, and most preferably wherein the labelling molecule is a fluorescently or quantum dot labelled molecule.
65. The method of any of the preceding embodiments, wherein the presence of the label molecule is measured by a cell analyser, preferably by flow cytometry or by microcapillary cytometry, more preferably by flow cytometry.
66. The method of any of the preceding embodiments, wherein the labelling molecule is a protein or an aptamer.
67. The method of embodiment 66, wherein the labelling molecule is a protein, preferably an antibody, nanobody or camelid single domain antibody, preferably wherein the labelling molecule is an antibody, more preferably a fluorescently labelled antibody.
68. The method of embodiment 66, wherein the labelling molecule is an aptamere.
69. The method of any of the preceding embodiments, wherein the label is present if the signal of the label is at least 2-fold higher than the negative control, preferably, preferably 3-fold, more preferably 4-fold, even more preferably 5-fold, even more preferably 6-fold, even more preferably 7-fold, even more preferably 8-fold, even more preferably 9-fold, even more preferably 10-fold, even more preferably 12-fold, even more preferably 15-fold, even more preferably 20-fold, and at most 1000-fold.
70. The method of any of the preceding embodiments, wherein the complex of step a) has been obtained by following the steps of:
   i. activating the catechol acid (B; benzene-1,2-diol acid);
   ii. linking the activated catechol acid (B) to the superparamagnetic iron-based particle (A), and
   iii. reacting said activated catechol acid with an amino group or the thiol group of a bait molecule (C).
71. The method of any of the preceding embodiments, wherein 5-20 times of the iron weight amount of A is linked to one weight amount of the bait molecule, preferably 7-15 times, more preferably, 8-13 times, even more preferably 9-11 times, and most preferably around 10 times.
72. The method of any of the preceding embodiments, wherein 10-1000 ug iron of A is linked to 1-100 ug of C, preferably wherein 20-500 ug iron of A is linked to 2-50 ug of C, more preferably wherein 40-200 ug iron of A is linked to 4-20 ug of C, even more preferably wherein 60-160 ug iron of A is linked to 6-16 ug of C, even more preferably wherein 80-130 ug iron of A is linked to 8-13 ug of C, and most preferably wherein around 100 ug iron of A is linked to around 10 ug of C.
73. The method of any of the preceding embodiments, wherein the complex consists of a superparamagnetic iron-based particle (A), catechol acid moieties (B), and bait molecules (C).
74. The method of any of the preceding embodiments, wherein the amino group or the thiol group of bovine serum albumin (BSA) is covalently bound to the complex of step (b) instead of the bait molecule and thereby serves a negative control.
75. A complex comprising,
   (A) a superparamagnetic iron-based particle,
   (B) one or more catechol acid moieties, and
   (C) one or more bait molecules,
   wherein B has been bound to A; and wherein B has been covalently linked to C by activating the catechol acid (benzene-1,2-diol acid) and reacting said activated catechol acid with amino groups or the thiol group of C.
76. The complex of embodiment 75, wherein the bait molecule is defined in any of embodiments 22-37, the superparamagnetic iron-based particles are defined in any of embodiments 40-47, the catechol acid is defined in any of embodiments 48-54, and/or the amino group or the thiol group of the protein of interest is defined in embodiment 21.
77. The complex of embodiments 75 or 76, wherein additionally a protein of interest is bound to the bait molecule, preferably wherein the protein of interest is further defined in any of embodiments 2-20.
78. The complex of any of embodiments 75-77, wherein the protein of interest is an antibody and/or wherein the bait molecule is an antibody.
79. The complex of any of embodiments 75-78, wherein the complex consists of a superparamagnetic iron-based particle (A), catechol acid moieties (B), and bait molecules (C).
80. The complex of any of embodiments 75-79, wherein B has been bound to A by a ligand exchange reaction.
81. The complex of any of embodiments 75-80, wherein A has been linked to a plurality of catechol acid moieties (B).
82. The complex of any of embodiments 75-81, wherein the plurality of catechol acid moieties (B) is linked to a plurality of bait molecules (C).
83. A method for diagnosing a disease or disorder, wherein a protein of interest is identified in a body fluid sample of a patient, the method comprising the steps of:
   a) providing a complex comprising
      (A) a superparamagnetic iron-based particle,
      (B) one or more catechol acid moieties, and
      (C) one or more bait molecules,
         wherein B has been bound to A; and wherein B has been covalently linked to C by activating the catechol acid (benzene-1,2-diol acid) and reacting said activated catechol acid with the amino group or the thiol group of C;
   b) contacting the complex of step a) with the sample whereby the bait molecule (C) binds to the protein of interest;
   c) immobilizing the complex binding to the protein of interest of step b) by a magnetic field and thereby separating the protein of interest from the sample,
   d) contacting the complex binding to the protein of interest of step c) with a detection molecule, wherein the detection molecule binds to the protein of interest; wherein the detection molecule (i) comprises a detectable label or (ii) is detectable by a labelling molecule comprising the label,
   e) determining the presence of the label, thereby identifying the protein of interest.
84. The method of embodiment 83, wherein the presence of the protein of interest indicates that the patient has a risk of developing or suffers from said disease or disorder.
85. The method of embodiments 83 or 84, wherein the disease or disorder is an infectious disease or auto-immune disorder, preferably wherein the disease or disorder is an infectious disease.
86. The method of any of embodiments 83-85, wherein the disease or disorder is caused by a virus, bacterium, fungus or protozoan.
87. The method embodiment 86, wherein the virus, bacterium, fungus or protozoan is selected from a pathogen as defined in any of embodiments 3-10 and 15-19.
88. The method of any of embodiments 83-87, wherein the protein of interest is defined in any of embodiments 2-20, the sample is defined in any of embodiments 38-39, the superparamagnetic iron-based particles are defined in any of embodiments 40-47, the catechol acid is defined in any of embodiments 48-54, the amino group or the thiol group of the protein of interest is defined in embodiment 21, and/or the bait molecule is defined in embodiment 22-37.
89. A method of preparing a complex, the method comprising the steps of:
   I. providing a superparamagnetic iron-based particle (A),
   II. activating catechol acid (B; benzene-1,2-diol acid);
   III. linking the activated catechol acid (B) to A, and
   IV. reacting said activated catechol acid with an amino group or the thiol group of a bait molecule (C).
90. The method of embodiment 89, wherein the superparamagnetic iron-based particles are defined in any of embodiments 40-47, the catechol acid is defined in any of embodiments 48-54, and/or the bait molecule is defined in any of embodiment 22-37.
91. Use of a complex for identifying a protein of interest in an aqueous or body fluid sample, wherein a comprises
   (A) a superparamagnetic iron-based particle,
   (B) one or more catechol acid moieties, and
   (C) one or more bait molecules,

   wherein B has been bound to A; and wherein B has been covalently linked to C by activating the catechol acid (benzene-1,2-diol acid) and reacting said activated catechol acid with an amino group or the thiol group of C;
   and wherein the protein of interest is capable of binding to the bait molecule.
92. The use of embodiment 91, wherein the protein of interest is defined in any of embodiments 2-20, the sample is defined in any of embodiments 38-39, the superparamagnetic iron-based particles are defined in any of embodiments 40-47, the catechol acid is defined in any of embodiments 48-54, the amino group or the thiol group of the protein of interest is defined in embodiment 21, the bait molecule is defined in any of embodiments 22-37, and/or wherein the magnetic field is defined in embodiment 57.
93. Kit for identifying a protein of interest in an aqueous or body fluid sample, wherein the kit comprises a complex comprising:
   (A) a superparamagnetic iron-based particle,
   (B) one or more catechol acid moieties, and
   (C) one or more bait molecules,

   wherein B has been bound to A; and wherein B has been covalently linked to C by activating the catechol acid (benzene-1,2-diol acid) and reacting said activated catechol acid with the amino group or the thiol group of C;
   and wherein the protein of interest is capable of binding to the bait molecule
94. The kit of embodiment 93, wherein the protein of interest is defined in any of embodiments 2-20, the sample is defined in any of embodiments 38-39, the superparamagnetic iron-based particles are defined in any of embodiments 40-47, the catechol acid is defined in any of embodiments 48-54, and/or the amino group or the thiol group of the protein of interest is defined in embodiment 21.

### Description of the Figures

**Fig. 1****:** Activation of caffeic acid with pentafluorophenyl trifluoroacetate (PFP-TFA).
**Fig. 2****:** Iron oxide nanoparticles with a hybrid coating of citrate and pentafluorophenyl caffeate (CAF-PFP)

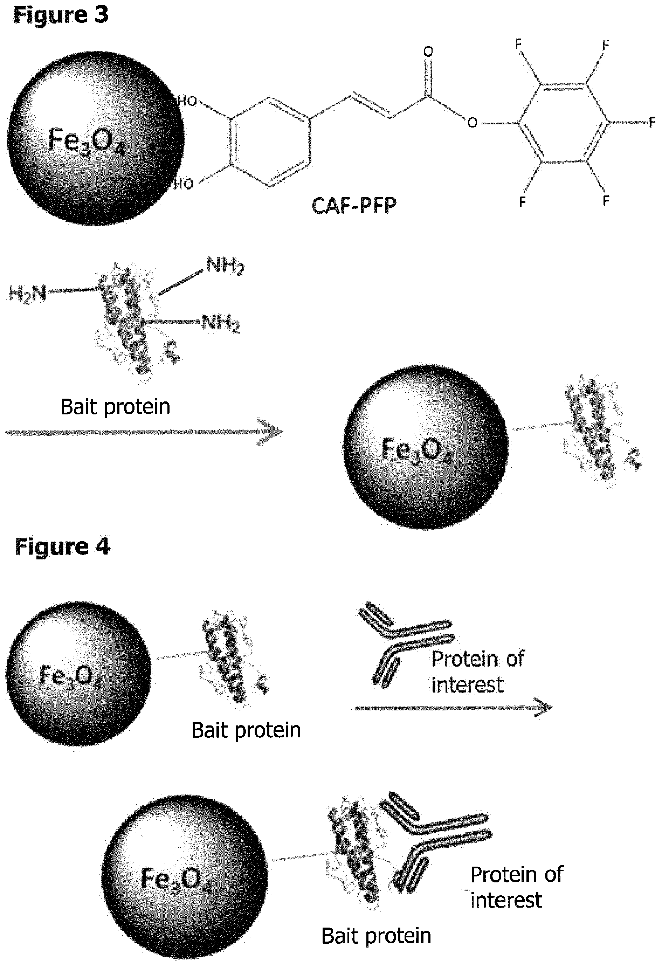

**Fig. 3****:** Binding of viral protein via correspondingly exposed amino groups to the surface of the iron oxide particles equipped with CAF-PFP.
**Fig. 4**: Detection of the protein of interest sought in the sample material.
**Fig. 5****:** Amid reaction (a) and Michael reaction (b) as cartoon representation.

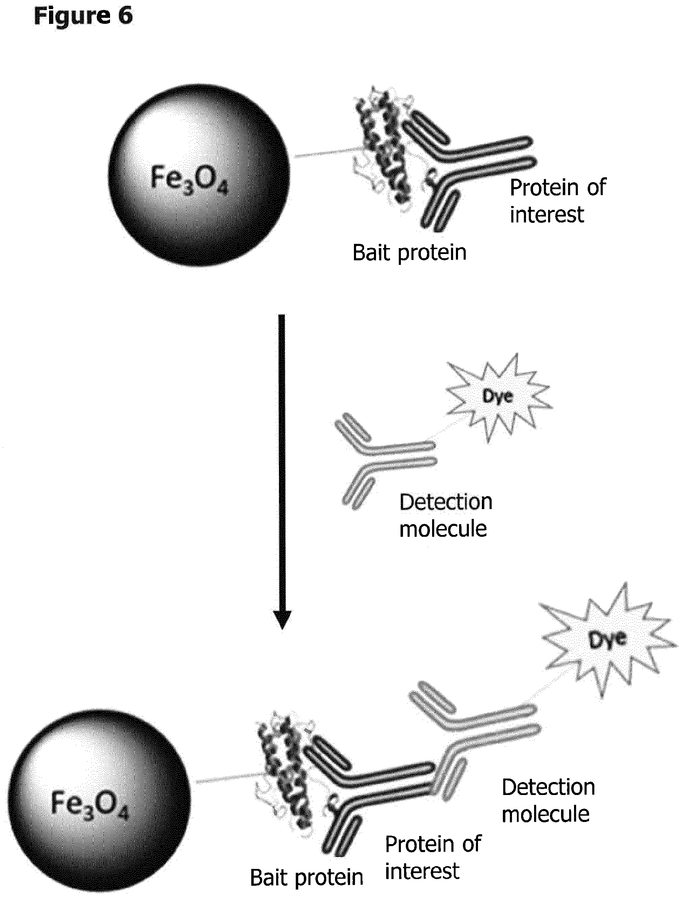

**Fig. 6****:** Labelling of the formed 'particle-bait protein-protein of interest' composite by a dye-labelled detection antibody that specifically binds to the protein of interest.

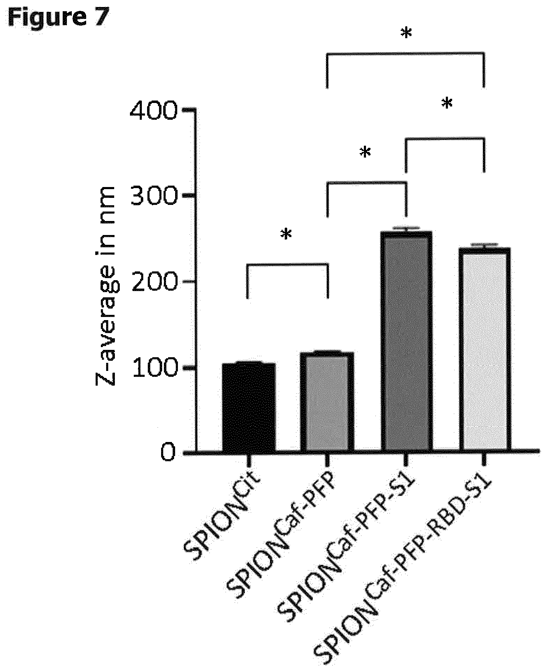

**Fig. 7****:** Z-average in nm of the different nanoparticles. Starting with citrate coated particles SPIONCit (103 nm) the functionalisation with/binding of CAF-PFP (SPION^{CAF-PFP}) results in a size increase to 117 nm. Size progression to SPION^{CAF-PFP} to the particles that bound S1 protein or the RBD domain of Sars-CoV-2 as antigen (SPION^{CAF-PFP-S1}; SPION^{CAF-PFP-RBD} S1) yields in size increases of 256 nm and 238 nm, respectively.

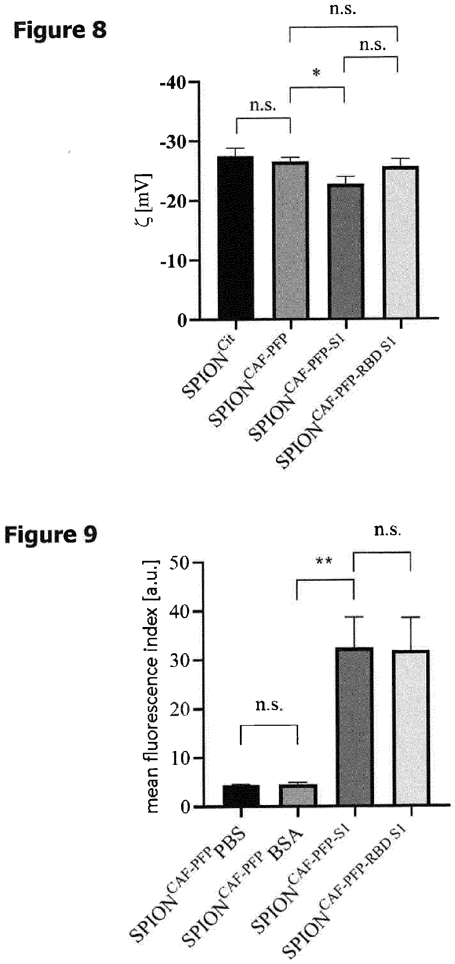

**Fig. 8****:** The development of the zeta potential behaves inconsistently. The zeta potential varies from - 27.4 mV to -26.5 mV and additional immobilisation of components in the particle shell are not reflected in zeta potential changes.
**Fig. 9**: Control 1 (SPION^{CAF-PFP}): SPIONs only modified with the linker molecule at the surface. Control 2 (SPION^{CAF-PFPBSA}): SPIONs with linker bound BSA; probe 1 (SPION^{CAF-PFP-S1}): SPIONs with linker bound Sars-CoV2-S1 (SinoBiological, Germany). Sample 2 (SPION^{CAF-PFP-RBD}): SPIONs with the linker bound RBD (receptor binding domain) (SinoBiological, Germany) of the Sars-CoV2-S1-protein.
**Fig. 10****:** Determination of anti-Sars-IgG-antibodies in three human patient sera using FACS-analysis. Panel 1 (control-panel): SPION^{CAF-PFP-particles}, SPION^{CAF-PFP-BSA-particles} and SPION^{CAF-PFP-S1-particles} incubated with patient sera 1-3 and the detection antibody directed against the mouse antibody from the *in vitro* experiments in **Fig.** 9. Panel 2: Incubation of the three particle types with the three patient sera and an antibody directed against human IgG-antibodies. Positively detected sera are serum1 and serum 2. Serum 3 was correctly identified as a negative sample.
**Fig. 11****:** FACS measurement: binding of ENV antigen (ENV=bright columns) or BSA as control (dark column) and labelling reaction with the 2G12 antibody. The left pair of columns shows the fluorescence base signal that is generated when no antibody is added. Addition of the antibody shows specific binding of the complementary antibody in the form of a highly significant increased fluorescence signal. The following sequence of labelling is present in the left column of the right pair of columns: SPION^{CAF-PFP}+ ENV antigen + antibody. In the right column, on the other hand, this labelling sequence is present: SPION^{CAF-PFP}+ BSA + antibody. Here, the antibody is only adsorbed non-specifically.

### Examples

The following examples are intended to illustrate the invention further, but are not limited to it. The examples describe technical features, and the invention also relates to combinations of the technical features presented in this section.

### Example 1

### Covalently Binding SARS-CoV-2 Antigen to SPION^{CAF-PFP}

In this Example, the covalent binding of SARS-CoV-2 Antigen to SPION^{CAF-PFP} is described. The term 'functionalization' of a particle means the binding of e.g. the activated catechol acid or the antigen to the particle. The CAF-PFP functionalized particles are suitable to bind exposed amino groups in the SARS-CoV2 S1 spike or just the RBD of the spike-protein target by their activated ester function (3). The following procedure was applied. Before covalently binding the antigen, the SPION^{CAF-PFP} was washed two times with 0,1 M borate buffer (pH 8).

Therefore, 200 µl buffer and 70 µl of a 1 mg Fe/ml dispersion of SPION^{CAF-PFP} was mixed and afterwards magnetically separated. For the coating with S1-spike-Protein or RBD-spike-protein, particles were redispersed in 140 µl puffer and an amount of 70 µl of the respective protein in PBS was added to reach a protein concentration of 5 µg/ml. As controls in the following tests the viral proteins were replaced by PBS or BSA with 5 µg protein/ml. The samples were then homogenized on a shaker for two hours at 700 rpm at 25 °C. After the covalent binding of the antiben, particles were magnetically separated and magnetically washed two times with PBS containing 0.1% BSA. Finally, the virus protein exposing particles (SPION-CAF-PFP-PROTᵥ) were dispersed again in 200 µl of sterile FACS-buffer containing PBS with 0.1% BSA and 0.02% sodium azide.

### Determination of Antibody binding ability of S1/S1 receptor binding domain (RBD) functionalized SPION^{CAF-PFP}

### Protein of interest capturing and detection

Next steps are the immobilization of the corresponding protein of interest **(****Fig.** 4) and finally detecting the protein of interest by a fluorescently labeled detection antibody.

An amount of 100 µl FACS-buffer added to 10 µl of the as prepared SPION-^{CAF-PFP-S1} or SPION-^{CAF-PFP-S1RBD} was deployed for each sample in a 96-V-bottom-plate and centrifuged down for 5 minutes at 3200 rcf. Supernatants were depleted and particles were well redispersed with 25 µl of a 10 wt%. BSA containing PBS solution was added and incubated for 15 minutes at 4 °C. Without any further washing an amount of 25 µl of SARS-CoV2-Spike-Sl antibody (diluted 1:200 in PBS) was added and incubated for 1 hour at 4 °C. An amount of 100 µl of FACS-buffer was added to each well followed by a centrifugation and depletion step as described before. To each well 100 µl of FACS-buffer were added and the washing step was repeated. After washing, 25 µl of the detection antibody (diluted 1:200 in PBS) were pipette to each well redispersing the particles. The plate was than incubated for another hour at 4 °C followed by two more washing steps. After the final centrifugation, the supernatant was completely depleted and the particles were redispersed in 100 µl FACS-buffer and transferred to FACS-tubes. The samples were analyzed by flow cytometry (Gallios, Beckman Coulter). As excitation wavelength 488 nm was set. Fluorescence bleed through was eliminated by electronic compensation. The acquired data was analyzed with the Kaluza software version 2.0 (Beckman Coulter).

### Determination of SARS-CoV-2-Antibodies in blood sera using S1/S1 RBD functionalized SPION^{CAF-PFP}

To test if SPION-^{CAF-PFP-S1} or SPION-^{CAF-PFP-S1RBD} are able to specifically binding antibodies from patient sera, the inventors used serum that did contain antibodies with high or low amount and without antibodies against SARS-COV-2. The amount was tested previously via Eclesis test from Roche [9].

Similar to the experiment with commercial antibodies an amount of 100 µl FACS-buffer was added to 10 µl of the as prepared SPION-^{CAF-PFP-S1} or SPION-^{CAF-PFP-S1RBD} was deployed for each sample in a 96-V-bottom-plate and centrifuged down for 5 minutes at 3200 rcf. Supernatants were depleted and particles were well redispersed with 25 µl of a 10 wt%. BSA containing PBS solution was added and incubated for 15 minutes at 4 °C. Similar to the previous test 25 µl of Serums (diluted 1:400 in PBS) were added directly and incubated for 1 hour at 4 °C. After the incubation 100 µl of FACS-buffer was added followed by a centrifugation and depletion step as described before. To each well 100 µl of FACS-buffer were added and the washing step was repeated. After washing, 50 µl of the detection antibody (diluted 1:500 in PBS) were pipetted to each well redispersing the particles. Two detection antibodies (IgG anti-human antibodies) were tested and as a control the IgG1 mouse isotype was used to make sure that no unspecific binding did occur. The plate was than incubated for another hour at 4 °C followed by two more washing steps. After the final centrifugation, the supernatant was completely depleted and the particles were redispersed in 100 µl FACS-buffer and transferred to FACS-tubes. The samples were analyzed by flow cytometry (Gallios, Beckman Coulter). As excitation wavelength 360 nm was set for these antibodies. Fluorescence bleed through was eliminated by electronic compensation. The acquired data was analyzed with the Kaluza software version 2.0 (Beckman Coulter).

### Results and Discussion

Synthesis of CAF-PFP pentafluorophenol caffeic acid ester gave yields of approx. 80% after purification, which was performed using Interchim PuriFlash 450 apparatus and deploying CHROMABOND Flash RS 40 cartridges. Crude Caf-PFP sample was dissolved in EtOAc/hexane 4/6 mixture and separated in two portions. Each one of them was loaded on cartridge (CHROMABOND RS 40) and eluted with EtOAc/hexane mixture with total flow rate of 26 ml/min. Peak with retention time of 4 minutes was collected, solvent was removed in vacuo to give Caf-PFP as colorless oil. (Rf = 0.6). NMR spectra were acquired on a Bruker Avance 300 or a Bruker Avance 400 spectrometer.

1H-NMR (300 MHz, acetone-d6): δ (ppm) = 8.93-8.11 (br. s., 1H), 7.91 (d, J = 15.8 Hz, 1H), 7.33 (d, J = 2.1 Hz, 1H), 7.24 (dd, J = 8.3 Hz, J = 2.1 Hz, 1H), 6.95 (d, J = 8.2 Hz, 1H), 6.62 (d, J = 15.8 Hz, 1H);

19F-NMR (282.53 MHz, acetone-d6): δ (ppm) = -155.2 (m, 2F), -160.9 (t, J = 21.3 Hz, IF), 165.1 (t, J = 20.3 Hz, 2F);

13C-NMR (100.68 MHz, acetone-d6): δ (ppm) = 163.7, 151.0, 150.1, 146.5, 143.5, 141.2, 140.1, 137.6, 126.9, 123.9, 116.5, 115.9, 111.0;

Synthesis of the citrate-coated particles leads to particle sizes of 103 nm and increases to 117 nm hydrodynamic diameter after functionalization with CAF-PFP. Further immobilization with the S1 antigen or the RBD domain of the S1 protein leads to size increases of 256 and 238 nm, respectively. In contrast, the development of the zeta potential behaves inconsistently (Tab 1) **(Fig., Fig.** ).

**Table 1: Z-average, PDI and zetapotenial of used nanoparticles**

| **SPION^{CIT}** | **SPION^{CAF-PFP}** | **SPION^{CAF- PFP-S1}** | **SPION^{CAF-PFP-S1 RBD}** |
|---|---|---|---|
| Z-average in nm 103 | 117± 1.0 | 256 ± 4.5 | 237.6 ± 3.6 |
| PDI | 0.15 | 0.473 | 0.425 |
| ζ [mV] | - 26 ± 1.26 | - 22.7 ± 0.58 | - 25.6 ± 1.08 |

Controls and samples were incubated with anti-SARS-Cov antibody (Chimeric MAb (mouse/human) SARS-CoV/Sars-CoV2 Spike, SinoBiological, Germany) with reactivity against Sars-CoV2 S1 and Sars-CoV2 S1-RBD and the dye labelled detection antibody (Donkey anti-mouse IgG (H+L) Alexa Fluor 488, Thermo Fisher Scientific, Germany) directed against the primary antibody, as indicated in **Fig..** Flow cytometry data revealed only background fluorescence in controls 1 and 2. In contrast, a high signal could be detected in samples one and two after sequential incubation with both antibodies. The signal difference between controls and samples was highly significant (p < 0.0001). The differences between the controls as well the samples themselves were not significant.

The mouse antibody in the control panel did not induce a significant signal above the background **(****Fig.** 10). In the second panel, in two (serum 1 and 2) of three patient sera anti-human-IgG antibodies could be caught by the bait molecule bound particles (also called: antigen functionalized particles, column 11 and 12). Particles, which were only blocked with BSA (column 8,9 and 10) were not able to ligate the anti-human-IgG antibodies in a way which is significantly higher than non-specific adsorption. Non-specific adsorption, which was detectable for the detection antibody was also much below the level of selectively labelling of previously captured anti-human-IgG antibodies (column 14). In conclusion, the signal of the two confirmed Sars-CoV2 positive sera was significantly higher than the background and the samples with random binding, whereas the signal of the sample with known negative Sars-CoV2-status did not exceed the background signal.

Hence, the data revealed a correct determination of patient sera having a positive or negative Sars-CoV2 status.

### Example 2 - Assay of the HIV 1 ENV Antigen—2G12 antibody complementary binding pair

The versatility of the principle becomes clear by transferring it to another complementary binding pair. This consists of an HIV surface protein as bait protein **(ENV** UG37gp140 (clade A), Polymun Scientific, Klosterneuburg, Austria) and the corresponding protein of interest (antibody, **2G12:** recombinant human monoclonal antibody to HIV-1 gp120; Ab002, Polymun Scientific, Klosterneuburg, Austria) which is already labelled with Alexa^{™} 647 as a fluorescent dye. Therefore, no further antibody is needed in this setting. 175 µl SPION^{CAF-PFP} Suspension at a concentration of 1 mg/ml was added. 25 µl **ENV** (conc.: 0.4 µg/µl), or bovine serum albumin in the same concentration as negative control were added and incubated for 2h at 700 rpm. This is followed by centrifugation and removal of 200 µl supernatant. Then adding 100 µl PBS and redispersing. After that, 100 µl supernatant are taken. The result is the antigen-functionalised SPION^{CAF-PFP-ENV} or the SPION^{CAF-PFP-BSA} control. In the next step, 80 µl of **2G12** antibody diluted in PBS is added to the redispersed SPIONS^{CAF-PFP-GP120} (or the SPION^{CAF-PFP-BSA)} and incubated for 1h at 4°C under light protection. This is followed by 10 minutes of magnetic separation and the removal of 80 µl supernatant. Then 100 µl PBS was added and the particles redispersed again. This is followed by another 10-minute magnetic separation and the removal of 100 µl supernatant. Now 175 µl of PBS was added and after further redispersion the measurement was carried out in the flow cytometry (Gallios, Beckman Coulter). As excitation wavelength 360 nm was set for these antibodies. Fluorescence bleed through was eliminated by electronic compensation. The acquired data was analyzed with the Kaluza software version 2.0 (Beckman Coulter) (Fig.). In detail, the underlying experimental design was as follows: Column 1: SPIONs with ENV protein. Column 2: SPIONs with BSA pre-blocking followed by incubation with ENV protein. Column 3: SPIONs with ENV protein followed by incubation with dye-labelled 2G12. Column 4: SPIONs with BSA pre-blocking followed by incubation with ENV protein and finally incubation with dye labelled-2G12.

In summary, **Fig.** 11 shows that the envelope specific antibody readily bound to the bait protein and thus the protein of interest could be readily identified in the sample.

### Discussion

In principle, the method is widely applicable, as it is a stochastic binding that addresses exposed amino groups or thiol groups in the target. In the diagnostic environment, a wide variety of antigens can be immobilized here, which can subsequently be detected by complementary antibodies. Using a fluorescent dye-labeled antibody as a detection molecule directed against the protein of interest, reliable values can be obtained e.g. by flow cytometry.

### Materials and Methods

### Materials

SARS-CoV2 S1-Spike-Protein (His Tag), Sino Biological, Cat-No.: 40591-V08H; SARS-CoV2-Spike-Sl Antibody, Chimeric Mab, Sino Biological, Cat-No.: 40150-D001; Goat-anti-human IgG Alexa Flour 488 donkey anti mouse, Phosphate-buffered saline (PBS) were purchased from Sigma-Aldrich, BSA, Natriumazid, boric acid were purchased from Carl Roth GmbH + Co, KG, NaOH. Bromoacetic acid was purchased from Alfa Aesar. The water used for all experiments came from a Siemens Ultra Clear system (Evoqua Water Technologies, Guenzburg, Germany). Detection in human serum via detection antibody (anti-human IgG Antibody, anti-human, VioBlue; order no: 130-119-967); control antibody 130-113-205: Isotyp-control, mouse IgG1, VioBlue; Miltenyi Biotech GmbH, Bergisch Gladbach, Germany.

### Synthesis of Activated Caffeic Acid

The derivatization of CAF-PFP **(****Fig.** 1) is essentially based on the work of Williams et al.[7] (Supplementary Information), but slightly adapted. In brief: 790.2 mg caffeic acid are dissolved in 10 ml dimethylformamid. This is followed by the addition of 570 µl pyridine and then the addition of 1.21 ml pentafluorophenyl trifluoroacetate. The mixture is stirred for 2h at RT (300 rpm) and then diluted with 30 ml dichloromethane and transferred to a separation funnel. There it is washed 5 times with 25 ml 1 N HCI. The remaining organic phase is dried with anhydrous magnesium sulphate and finally reduced on a rotary evaporator. This is followed by chromatographic purification with silica gel 60 grain size 0.04-0.63 mm (Carl Roth GmbH order no. P091.2). The product has an R-f value of 0.64 and is isolated accordingly and concentrated to dryness on the rotary evaporator. An alternate route for purification is using Interchim PuriFlash 450 apparatus and deploying CHROMABOND Flash RS 40 cartridges. Crude Caf-PFP sample was dissolved in EtOAc/hexane 4/6 mixture and separated in two portions. Each one of them was loaded on cartridge (CHROMABOND RS 40) and eluted with EtOAc/hexane mixture with total flow rate of 26 ml/min. Peak with retention time of 4 minutes was collected, solvent was removed in vacuo to give Caf-PFP as colorless oil. (Rf = 0.6).

### CAF-PFP particles:

The previously generated CAF-PFP active ester must first be bound to the particle surface. This functionalization is carried out in the form of a ligand exchange reaction with particles previously coated with citrate **(****Fig.** 2).

Citrate-stabilized SPIONs were synthesized by a simple co-precipitation method described in our previous work [8]. In short, an aqueous solution of iron (II) and iron (III) salts containing 13.2 mg iron/ml is deoxygenized using argon. The precipitation of SPIONs is achieved by injecting 25% NH_{3 (aq)} into the vigorously stirring iron salt solution. After stirring the darkened SPION dispersion at 400 rpm for 10 min at room temperature, 15 mL of a 293.3 mg/mL sodium citrate solution was added and the temperature was raised to 90 °C. The dispersion was further stirred for 30 min, before letting it cool to room temperature and transferring it into 10 kDa dialysis tubes. The dispersion was dialyzed for 6 h against H₂O. Once the dialysis was done, the SPIONs were filtered through a 0.2 µm filter membrane (Minisart^{®}, Sartorius AG, Göttingen, Germany) to achieve the final citrate-stabilized SPIONs. The resulting particles are present as a stable aqueous suspension and have a hydrodynamic size of 99 nm and are ready for the subsequent CAF-PFP functionalization. 2 mg CAF-PFP are dissolved in 1 ml acetonitrile. The citrate-coated particles are dispersed in acetate buffer at pH 4 (0.1 M) at a concentration of 2 mg Fe/ ml. To 1 ml of particle dispersion, 2 ml of acetate buffer is added and then 1 ml of CAF-PFP solution is added. The reaction mixture is stirred at RT overnight (250 rpm). To remove excess CAF-PFP, the mixture is centrifuged with 6000 rcf (Centrifuge 5430 R, Eppendorf AG, Hamburg, Germany) the supernatant is discarded and the residue is taken up again with water/acetonitrile (1:1). This washing step is repeated once, agitating the pellet in an ultrasonic bath for 5 min each time (Bandelin Sonorex Digitec, Bandelin electronic GmbH & Co KG, Berlin, Germany). After the last centrifugation the residue is redispersed again in bidest. water.

The particles functionalized with CAF-PFP are therefore stably suspended in aqueous medium, since there is still a proportion of citrate on the particle surface that has not been substituted by CAF-PFP.

### References

1. Sarcletti, M.; Vivod, D.; Luchs, T.; Rejek, T.; Portilla, L.; Muller, L.; Dietrich, H.; Hirsch, A.; Zahn, D.; Halik, M., Superoleophilic Magnetic Iron Oxide Nanoparticles for Effective Hydrocarbon Removal from Water. Adv Funct Mater 2019, 29(15).
2. Etelka, T.; Marta, S.; Angela, H.; Ildiko, Y. T.; Rita Andrea, B.; Daniel, N.; Erzsebet, I.; Istvan, Z.; Ladislau, V., Colloidal stability of carboxylated iron oxide nanomagnets for biomedical use. Period Polytech-Chem 2014, 58, 3-10.
3. Tombacz, E.; Toth, I. Y.; Nesztor, D.; Illes, E.; Hajdu, A.; Szekeres, M.; Vekas, L., Adsorption of organic acids on magnetite nanoparticles, pH-dependent colloidal stability and salt tolerance. Colloid Surface A 2013, 435, 91-96.
4. Abugazleh, M. K.; Rougeau, B.; Ali, H., Adsorption of catechol and hydroquinone on titanium oxide and iron (III) oxide. J Environ Chem Eng 2020, 8(5), 104180.
5. Amstad, E.; Gillich, T.; Bilecka, I.; Textor, M.; Reimhult, E., Ultrastable Iron Oxide Nanoparticle Colloidal Suspensions Using Dispersants with Catechol-Derived Anchor Groups. Nano Lett2009, 9(12), 4042-4048.
6. Chen, T.-P.; Liu, T.; Su, T.-L; Liang, J., Self-Polymerization of Dopamine in Acidic Environments without Oxygen. Langmuir 2017, 33(23), 5863-5871.
7. Williams, L. K.; Zhang, X.; Caner, S.; Tysoe, C.; Nguyen, N. T.; Wicki, J.; Williams, D. E.; Coleman, J.; McNeill, J. H.; Yuen, V.; Andersen, R. J.; Withers, S. G.; Brayer, G. D., The amylase inhibitor montbretin A reveals a new glycosidase inhibition motif. Nat Chem Biol 2015, 11 (9), 691-696.
8. Stein, R.; Friedrich, B.; Muhlberger, M.; Cebulla, N.; Schreiber, E.; Tietze, R.; Cicha, I.; Alexiou, C.; Dutz, S.; Boccaccini, A. R.; Unterweger, H., Synthesis and Characterization of Citrate-Stabilized Gold-Coated Superparamagnetic Iron Oxide Nanoparticles for Biomedical Applications. Molecules 2020, 25(19).
9. Favresse, J.; Cadrobbi, J.; Eucher, C.; Elsen, M.; Laffineur, K.; Dogne, J.-M.; Douxfils, J., Clinical performance of three fully automated anti-SARS-CoV-2 immunoassays targeting the nucleocapsid or spike proteins. Journal of Medical Virology*.*
10. Wu Z., Van Ryk D., Davis C., Abrams W.R., Chaiken I., Magnani J., Malamud D. Salivary agglutinin inhibits HIV type 1 infectivity through interaction with viral glycoprotein 120, AIDS Research and Human Retroviruses, (2003)19(3), pp. 201-209.
11. Nieuwlandt D, Wecker M, Gold L. In vitro selection of RNA ligands to substance P. Biochemistry.1995; 34:5651-5659. [PubMed: 7537093]
12. Williams KP, Liu XH, Schumacher TN, Lin HY, et al. Bioactive and nuclease-resistant I-DNA ligand of vasopressin. Proc. Natl. Acad. Sci. USA. 1997; 94:11285-11290. [PubMed: 9326601]
13. Tuerk C, MacDougal S, Gold L. RNA pseudoknots that inhibit human immunodeficiency virus type 1 reverse transcriptase. Proc. Natl. Acad. Sci. USA. 1992; 89:6988-6992. [PubMed: 1379730]
14. Kubik MF, Stephens AW, Schneider D, Marlar RA, Tasset D. High-affinity RNA ligands to human α-thrombin. Nucleic Acids Res. 1994; 22:2619-2626. [PubMed: 7518917]
15. Somasunderam A, Ferguson MR, Rojo DR, Thiviyanathan V, et al. Combinatorial selection, inhibition, and antiviral activity of DNA thioaptamers targeting the RNase H domain of HIV-1 reverse transcriptase. Biochemistry. 2005; 44:10388-10395. [PubMed: 16042416]
16. Kang J, Lee MS, Watowich SJ, Gorenstein DG. Combinatorial selection of a RNA thioaptamer that binds to Venezuelan equine encephalitis virus capsid protein. FEBS Lett. 2007; 581:2497-2502. [PubMed: 17493617]
17. Urvil PT, Kakiuchi N, Zhou DM, Shimotohno K, et al. Selection of RNA aptamers that bind specifically to the NS3 protease of hepatitis C virus. Eur. J. Biochem. 1997; 248:130-138. [PubMed: 9310370]
18. Dadfar, S. M., Roemhild, K., Drude, N. I., von Stillfried, S., Knuechel, R., Kiessling, F., and Lammers, T. (2019) Iron oxide nanoparticles: Diagnostic, therapeutic and theranostic applications. Adv. Drug Delivery Rev. 138, 302-325
19. Lippi G, Salvagno G, Pegoraro M, Militello V, Caloi C, Peretti A, De Nitto S, Bovo C and Lo Cascio G. "Preliminary evaluation of Roche Cobas Elecsys Anti-SARS-CoV-2 chemiluminescence immunoassay" Clinical Chemistry and Laboratory Medicine (CCLM), vol. 58, no. 11, 2020, pp. e251-e253.
20. L. Zeininger, L. Portilla, M. Halik, A. Hirsch, (2016) Quantitative Determination and Comparison of the Surface Binding of Phosphonic Acid, Carboxylic Acid, and Catechol Ligands on TiO2 Nanoparticles. Chem. Eur. J. 22, 13506.
21. Mohammed, L., Ragab, D., and Gomaa, H. (2016) Bioactivity of Hybrid Polymeric Magnetic Nanoparticles and Their Applications in Drug Delivery. Curr. Pharm. Des. 22, 3332-3352
22. Drabik, A., Ner-Kluza, J., Mielczarek, P., Civit, L., Mayer, G., and Silberring, J. (2018) Advances in the Study of Aptamer-Protein Target Identification Using the Chromatographic Approach. J. Proteome Res. 17, 2174-2181
23. Yazdian-Robati, R., Ramezani, M., Khedri, M., Ansari, N., Abnous, K., and Taghdisi, S. M. (2017) An aptamer for recognizing the transmembrane protein PDL-1 (programmed death-ligand 1), and its application to fluorometric single cell detection of human ovarian carcinoma cells. Microchim. Acta 184, 4029-4035

## Claims

1. A method for identifying a protein of interest in an aqueous or body fluid sample, the method comprising the steps of:
a) providing a complex comprising, preferably consisting of,
(A) a superparamagnetic iron-based particle,
(B) one or more catechol acid moieties, and
(C) one or more bait molecules,
wherein B has been bound to A; and wherein B has been covalently linked to C by activating the catechol acid (benzene-1,2-diol acid) and reacting said activated catechol acid with an amino group or thiol group of C;
b) contacting the complex of step a) with the sample whereby the bait molecule (C) binds to the protein of interest;
c) immobilizing the complex binding to the protein of interest of step b) by a magnetic field and thereby separating the protein of interest from the sample,
d) contacting the complex binding to the protein of interest of step c) with a detection molecule, wherein the detection molecule binds to the protein of interest; wherein the detection molecule (i) comprises a detectable label or (ii) is detectable by a labelling molecule comprising the label,
e) determining the presence of the label,
thereby identifying the protein of interest.

2. The method of claim 1, wherein the catechol acid is caffeic acid, preferably wherein the caffeic acid has been activated to be a pentafluorophenyl caffeic acid, terafluorophenyl caffeic acid, or a caffeic acid N-Hydroxysuccinimide ester, preferably wherein the catechol acid has been activated by pentafluorophenyl trifluoroacetate.

3. The method of any of the preceding claims, wherein the protein of interest is a pathogen protein, preferably wherein the pathogen protein is a viral protein, a bacterial protein, a fungal protein, or a protozoan protein, preferably wherein the viral protein is from a coronavirus, more preferably wherein the coronavirus is a SARS-coronavirus or a MERS-coronavirus, even more preferably a SARS coronavirus, and most preferably SARS-coronavirus 2; or wherein the viral protein is from a lentivirus, more preferably from human immunodeficiency viruses (HIV); and/or
wherein the protein of interest is an antibody having been formed in response to an infection by a pathogen or a vaccination against said pathogen, preferably wherein the pathogen is a viral or bacterial pathogen, preferably wherein the viral pathogen is a coronavirus, preferably wherein the coronavirus is a orthocoronavirus, preferably a betacoronavirus, more preferably a sarbecovirus, even more preferably a SARS-coronavirus, and most preferably SARS-coronavirus 2.

4. The method of any of the preceding claims, wherein the bait molecule is a protein or an aptamere, preferably wherein the bait molecule is a pathogen protein, more preferably wherein the pathogen protein is a viral protein, preferably wherein the viral protein is from a coronavirus, more preferably wherein the coronavirus is a SARS-coronavirus or a MERS-coronavirus, even more preferably a SARS coronavirus, and most preferably SARS-coronavirus 2; or wherein the viral protein is from a lentivirus, more preferably from human immunodeficiency viruses (HIV); and/or
wherein the bait molecule is an antibody, preferably wherein the bait molecule is an antibody binding to a protein of interest as defined in claim 3.

5. The method of any of the preceding claims, wherein the aqueous or body fluid sample is a body fluid, waste water, ground water, or drinking water, preferably wherein the body fluid sample is a human body fluid, more preferably wherein the human body fluid is selected from blood, serum, plasma, lymph, urine, liquor, saliva and sputum, even more preferably wherein the human body fluid is blood or serum, and even more preferably wherein the human body fluid is blood.

6. The method of any of the preceding claims, wherein the superparamagnetic iron-based particles are selected from an iron oxide, iron (Fe), iron-cobalt, alnico, permalloy particles, preferably wherein the superparamagnetic iron-based particles are superparamagnetic ironoxide nanoparticles (SPIONs).

7. The method of any of the preceding claims, wherein the superparamagnetic iron-based particles are coated with citrate, oxalic acid, ascorbic acid, amino acids, preferably citrate.

8. The method of any of the preceding claims, wherein the detection molecule is a protein, preferably an antibody, nanobody or camelid single domain antibody, preferably wherein the detection molecule is an antibody; and/or
wherein the detection molecule comprises a detectable label, preferably wherein the detection molecule is covalently linked to a detectable label, more preferably wherein the detectable label is a fluorophore or a quantum dot, even more preferably wherein the detectable label is a fluorophore, even more preferably wherein the detection molecule is a fluorescently or quantumdot labelled molecule and even more preferably wherein the detectable label is a fluorescently labelled molecule.

9. The method of any of the preceding claims, wherein the complex of step a) has been obtained by following the steps of:
i. activating the catechol acid (B; benzene-1,2-diol acid);
ii. linking the activated catechol acid (B) to the superparamagnetic iron-based particle (A), and
iii. reacting said activated catechol acid with the amino group or the thiol group of a bait molecule (C).

10. A complex comprising,
(A) a superparamagnetic iron-based particle,
(B) one or more catechol acid moieties, and
(C) one or more bait molecules,
wherein A has been bound to B; and wherein B has been covalently linked to C by activating the catechol acid (benzene-1,2-diol acid) and reacting said activated catechol acid with an amino group or thiol group of C.

11. The complex of claim 10, wherein the catechol acid is defined in claim 2, the bait molecule is defined in claim 4, the sample is defined in claim 5, and/or the superparamagnetic iron-based particles are defined in claim 6.

12. A method for diagnosing a disease or disorder, wherein a protein of interest is identified in a body fluid sample of a patient, the method comprising the steps of:
a) providing a complex comprising
(A) a superparamagnetic iron-based particle,
(B) one or more catechol acid moieties, and
(C) one or more bait molecules,
wherein A has been bound to B; and wherein B has been covalently linked to C by activating the catechol acid (benzene-1,2-diol acid) and reacting said activated catechol acid with an amino group or thiol group of C;
b) contacting the complex of step a) with the sample whereby the bait molecule (C) binds to the protein of interest;
c) immobilizing the complex binding to the protein of interest of step b) by a magnetic field and thereby separating the protein of interest from the sample,
d) contacting the complex binding to the protein of interest of step c) with a detection molecule, wherein the detection molecule binds to the protein of interest; wherein the detection molecule (i) comprises a detectable label or (ii) is detectable by a labelling molecule comprising the label,
e) determining the presence of the label,
thereby identifying the protein of interest.

13. The method of claim 12, wherein the catechol acid is defined in claim 2, the protein of interest is defined claim 3, the bait molecule is defined in claim 4, the sample is defined in claim 5, the superparamagnetic iron-based particles are defined in claim 6, and/or wherein the detection molecule is defined in claim 7.

14. Use of a complex for identifying a protein of interest in an aqueous or body fluid sample, wherein a comprises
(A) a superparamagnetic iron-based particle,
(B) one or more catechol acid moieties, and
(C) one or more bait molecules,
wherein A has been bound to B; and wherein B has been covalently linked to C by activating the catechol acid (benzene-1,2-diol acid) and reacting said activated catechol acid with an amino group or thiol group of C;
and wherein the protein of interest is capable of binding to the bait molecule.

15. The use of claim 14, wherein the catechol acid is defined in claim 2, the protein of interest is defined claim 3, the bait molecule is defined in claim 4, the sample is defined in claim 5, and/or the superparamagnetic iron-based particles are defined in claim 6.
